(19) Europäisches Patentamt
European Patent Office
Office européen des brevets

(11) **EP 2 233 068 A1**

(12) **EUROPEAN PATENT APPLICATION**

(43) Date of publication:
**29.09.2010 Bulletin 2010/39**

(51) Int Cl.:
***A61B 5/00*** *(2006.01)*   ***A61B 5/151*** *(2006.01)*

(21) Application number: **10156880.6**

(22) Date of filing: **18.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **24.03.2009 JP 2009071684**

(71) Applicant: **SYSMEX CORPORATION**
**Kobe-shi,**
**Hyogo 651-0073 (JP)**

(72) Inventors:
• **Asakura, Yoshihiro**
**Kobe-shi, Hyogo 651-0073 (JP)**
• **Sato, Toshiyuki**
**Kobe-shi, Hyogo 651-0073 (JP)**
• **Okada, Seiki**
**Kobe-shi**
**Hyogo 651-0073 (JP)**
• **Tarutani, Jun**
**Tochigi 321-0202 (JP)**
• **Endo, Chika**
**Tochigi 321-0202 (JP)**

(74) Representative: **HOFFMANN EITLE**
**Patent- und Rechtsanwälte**
**Arabellastrasse 4**
**81925 München (DE)**

(54) **Puncture device and in vivo component measurement system**

(57)     A puncture device (1) which punctures a skin of a subject for extracting a tissue fluid from a puncture site of the subject. The puncture device included a timer (140); a notifying section which notifies a user that specific time measured by the time has passed; a puncture mechanism which punctuates the skin; and an interlock mechanism which caused the timer to start time measurement in conjunction with a punctuate action by the puncture mechanism.

FIG. 1

EP 2 233 068 A1

## Description

Field of the Invention

The present invention relates to a puncture device and an in vivo component measurement system.

Related Art

In order to measure a specific component such as glucose in a tissue fluid of a subject, for example, U.S. Patent Publication No. 2007/0233011 discloses a fine pore formation device which forms fine pores on a skin of a subject by puncturing the skin with a fine needle chip having many fine needles. According to this fine pore formation device, the glucose of the subject is measured in such a way that, after a puncture action, a tissue fluid is extracted from the subject's skin by contacting an extraction cartridge with a site of the puncture. In such measurement, time for extracting the tissue fluid has been previously set up and the subject has measured the time required for the extraction by using a watch or the like which is prepared separately.

However, there is a possibility that the subject forgets the measurement of the extraction time while conducting the puncture operation and the extraction operation or does not notice that the scheduled extraction has been completed.

## SUMMARY OF THE INVENTION

The scope of the present invention is defined solely by the appended claims, and is not affected to any degree by the statements within this summary.

In accordance with a first aspect of the present invention, there is provided a puncture device which punctures a skin of a subject for extracting a tissue fluid from a puncture site of the subject, comprising:

a timer;
a notifying section which notifies a user that specific time measured by the timer has passed;
a puncture mechanism which punctures the skin; and
an interlock mechanism which causes the timer to start time measurement in conjunction with a puncture action by the puncture mechanism.

Since the timer starts counting in conjunction with a puncture action by the puncture mechanism, there is no possibility of forgetting time measurement as conventional one. Further, since the notifying section notifies the subject that a specific time has passed, the subject is certainly able to notice that the scheduled extraction has been already completed. Therefore, it is possible to secure accurate extraction time which is necessary for measurement and obtain a highly reliable measurement result.

The notifying section may comprise at least one of an alarm sound generator and a vibrator.
The puncture device may further comprise a notification selecting section for selecting a notifying method by the notifying section.

The puncture device may further comprise a main body accommodating the puncture mechanism, and a timer unit removably mounted on the main body, and accommodating the timer and the notifying section.
The puncture device may further comprise a lock mechanism which inhibits the puncture action by the puncture mechanism when the timer unit has been removed from the main body.

The lock mechanism is preferably designed to allow the puncture action by the puncture mechanism when the timer unit has been mounted on the main body.
The puncture device may further comprise a press button which is pressed by the user for causing the puncture mechanism to start the puncture action, and the interlock mechanism may cause the timer to start the time measurement in conjunction with a pressing action of the press button by the user.

The puncture device may further comprise a memory, and a terminal for transferring from outside to the memory at least one of measurement information on measurement of a specific component in the tissue fluid of the subject and subject information on the subject, and for transferring at least one of the measurement information and the subject information which are memorized in the memory to outside.

The puncture device may further comprise a display section which displays at least one of remaining time before the specific time passes and clock time when the specific time passes.
The puncture device may further comprise a display selecting section for selecting an object to be displayed on the display section.

The puncture device may further comprise a turn-on mechanism which turns on a power supply of the display section in conjunction with an action of loading on the puncture mechanism a puncture needle for puncturing the skin.

In accordance with a second aspect of the present invention, there is provided an in vivo component measurement system comprising:

a puncture device which punctures a skin of a subject for extracting a tissue fluid from a puncture site of the subject, comprising:

a timer;
a notifying section which notifies a user that time measured by the timer has passed through the specific time;
a puncture mechanism which punctures the skin; and
an interlock mechanism which causes the timer to start time measurement in conjunction with a puncture action by the puncture mechanism;

a collection member for accumulating a specific component in the tissue fluid extracted from the puncture site punctured by the puncture device; and
a measurement device for obtaining a value related to a quantity of the specific component accumulated in the collection member.

The specific component may be glucose.
The measurement device may further comprise an analysis unit which obtains a value corresponding to an area under blood concentration - time curve (AUC) of the specific component during the specific time based on a value related to the specific components.
The collection member may comprise an extraction medium for accumulating the specific component in the tissue fluid and a retention member for retaining the extraction medium.

BRIEF DESCRIPTION OF THE DRAWINGS

FIG. 1 is a perspective view showing an overall configuration of an embodiment of a puncture device according to the present invention;
FIG. 2 is a perspective view showing an internal configuration of the puncture device shown in FIG. 1;
FIG. 3 is an exploded perspective view of the puncture device shown in FIG. 1;
FIG. 4 is a front view showing an internal configuration of a rear cover of the puncture device shown in FIG. 1;
FIG. 5 is a perspective view showing an internal configuration of a front cover of the puncture device shown in FIG. 1;
FIG. 6 is a bottom view of a chip accommodation tool insertion member of the puncture device shown in FIG. 1;
FIG. 7 is a front view of an array chuck of the puncture device shown in FIG. 1;
FIG. 8 is a perspective view of a release button of the puncture device shown in FIG. 1;
FIG. 9 is a perspective view showing an overall configuration of a chip accommodation kit provided with a fine needle chip to be mounted on the puncture device shown in FIG. 1;
FIG. 10 is an exploded perspective view of the chip accommodation kit shown in FIG. 9;
FIG. 11 is a perspective view of the fine needle chip of the chip accommodation kit shown in FIG. 9;
FIG. 12 is a sectional view taken along the line I-I of FIG. 10;
FIG. 13 is a top view of a chip accommodation tool of the chip accommodation kit shown in FIG. 9;
FIG. 14 is a perspective view of the chip accommodation tool of the chip accommodation kit shown in FIG. 9;
FIG. 15 is a bottom view of the chip accommodation tool of the chip accommodation kit shown in FIG. 9;
FIG. 16 is a sectional view taken along the line II-II of FIG. 13;
FTGs. 17A to 17D are explanatory plan views of a timer unit of the puncture device shown in FIG. 1;
FIGs. 18A to 18C are explanatory views of a bottom view and both side views of the timer unit of the puncture device shown in FIG. 1;
FIG. 19 is a view explaining a state in which the timer unit is mounted on a main body;
FIG. 20 is an explanatory view showing a state before the fine needle chip is mounted on the array chuck;
FIG. 21 is an explanatory view showing a state in which the array chuck mounted with a fine needle chip is moved to an ejectable position;
FIG. 22 is a schematic perspective view of a measurement device, a sensor chip, and a collection member which are used in a blood glucose AUC measurement method according to Example 1;
FIG. 23 is an explanatory plan view of the measurement device shown in FIG. 22;
FIG. 24 is an explanatory side view of the measurement device shown in FIG. 22;
FIG. 25 is an explanatory plan view of the sensor chip shown in FIG. 22;

FIG. 26 is an explanatory side view of the sensor chip shown in FIG. 22;

FIG. 27 is an explanatory sectional view of the collection member shown in FIG. 22;

FIG. 28 is a flowchart showing a measurement procedure of the blood glucose AUC measurement method according to Example 1;

FIG. 29 is an explanatory view of a measurement procedure of the blood glucose AUC measurement method according to Example 1;

FIG. 30 is an explanatory view of a measurement procedure of the blood glucose AUC measurement method according to Example 1;

FIG. 31 is an explanatory view of a measurement procedure of the blood glucose AUC measurement method according to Example 1;

FIG. 32 is an explanatory view of the blood glucose AUC measurement method according to Example 2;

FIG. 33 is an explanatory view of the blood glucose AUC measurement method according to Example 2;

FIG. 34 is an explanatory view of the blood glucose AUC measurement method according to Example 2; and

FIG. 35 is a block diagram of the timer unit.

DETAILED DESCRIPTION OF THE PREFERRED EMBODIMENTS

[Overall configuration of puncture device]

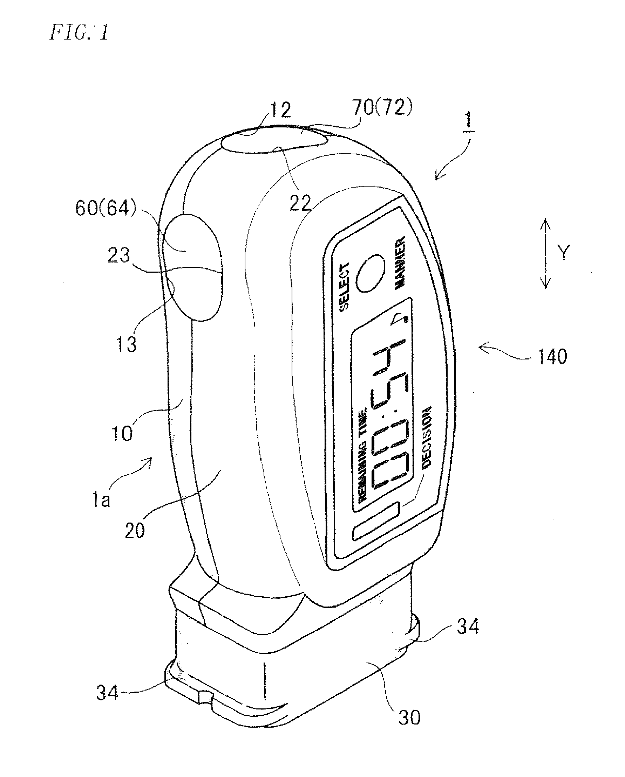

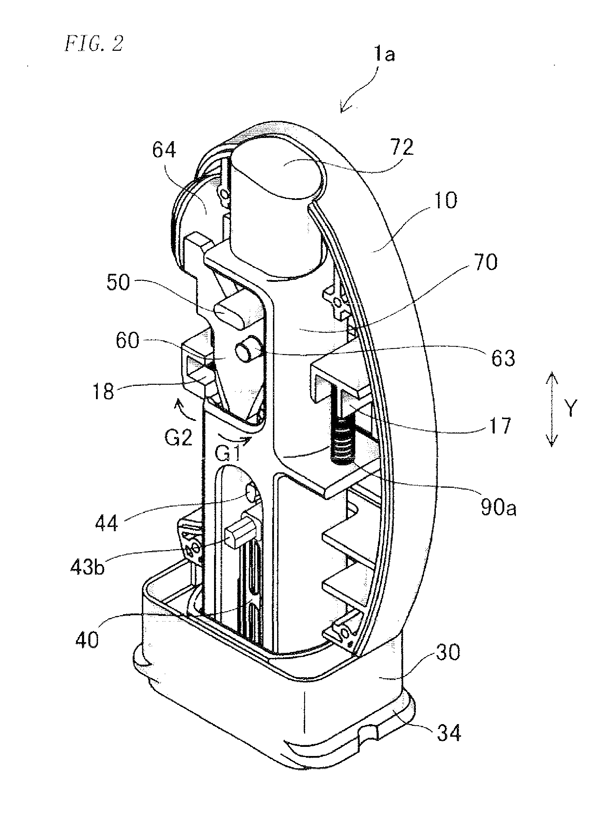

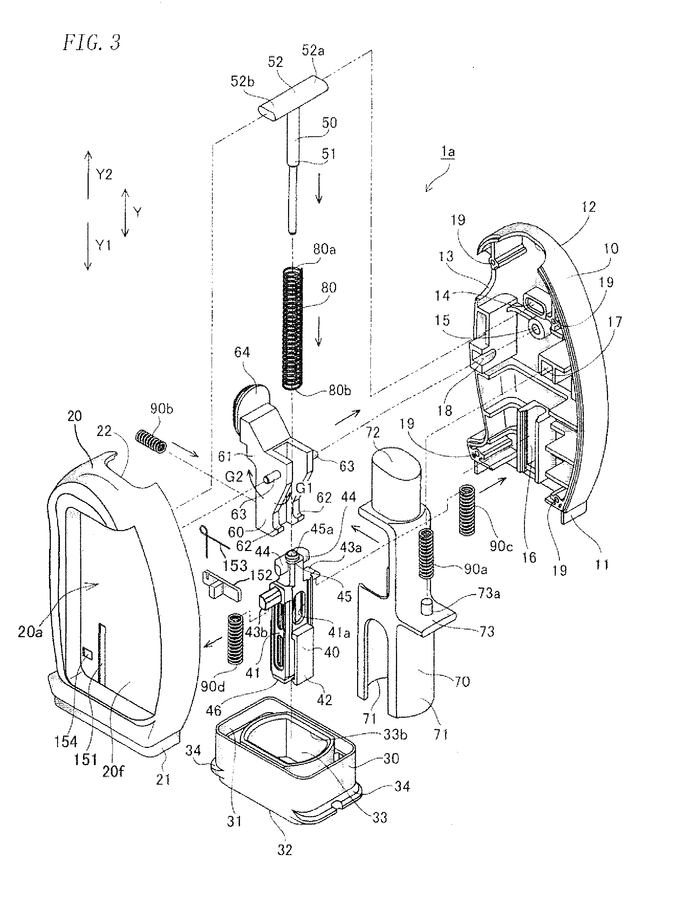

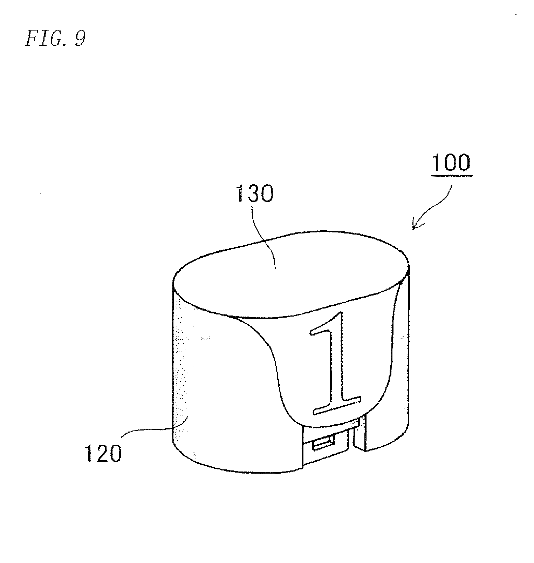

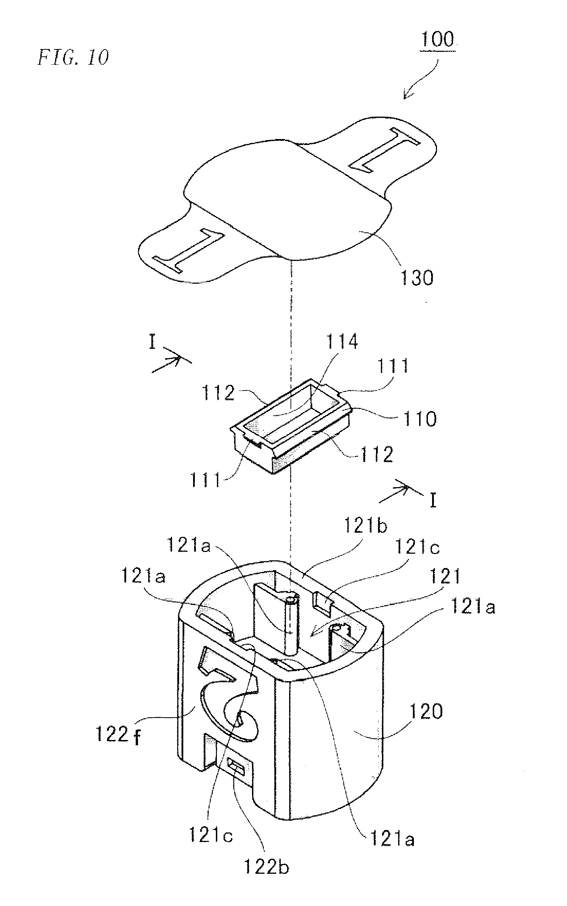

FIG. 1 is a perspective view showing an overall configuration of a puncture device 1 according to an embodiment of the present invention. FIGs. 2 to 8 are views for explaining detailed configuration of respective members of the puncture device 1 shown in FIG. 1. FIG. 9 is a perspective view showing an overall configuration of a chip accommodation kit provided with a fine needle chip to be mounted on the puncture device shown in FIG. 1. FIGs. 10 to 16 are views for showing a detailed configuration of respective members of the chip accommodation kit shown in FIG. 9. Here, although the timer unit is mounted on the main body in FIG. 1, the timer unit is removed from the main body for easy understanding in FIG. 3.

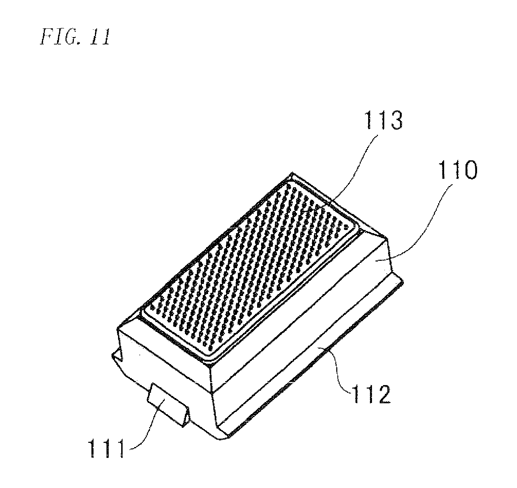

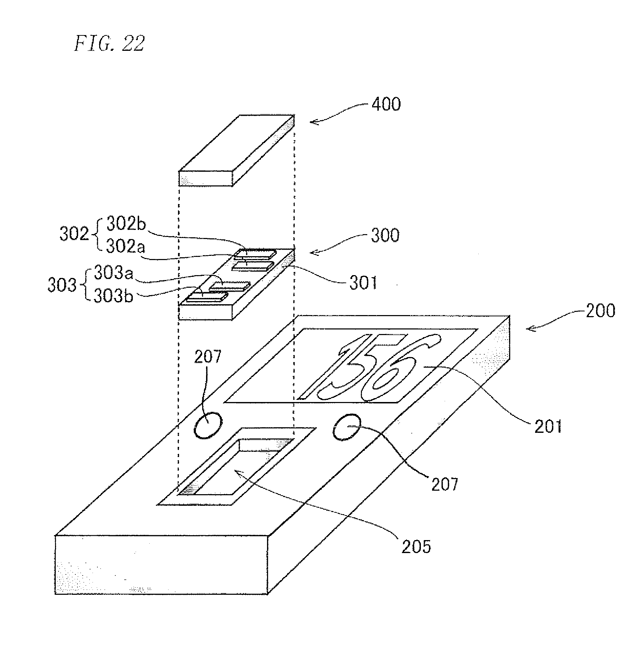

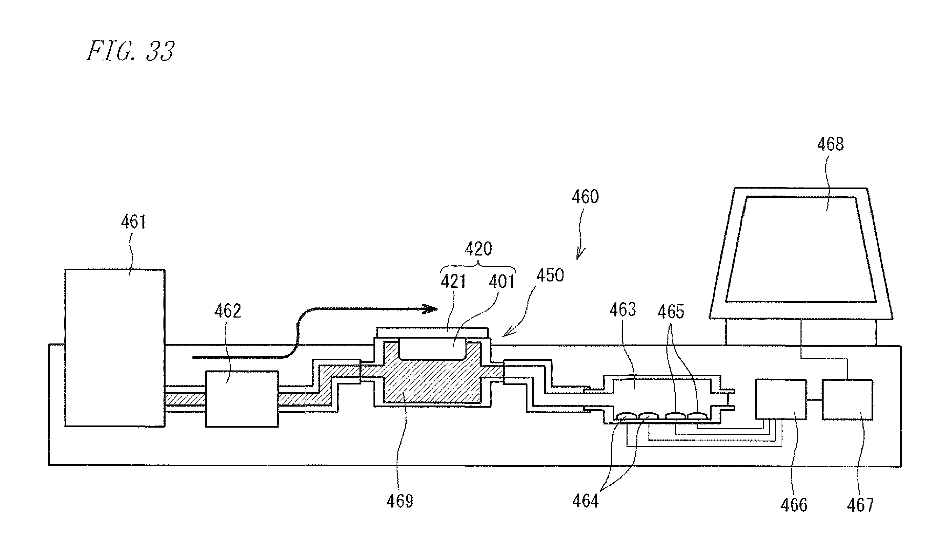

A puncture device 1 (Refer to FIG. 1) according to an embodiment of the present invention is mounted with a fine needle chip 10 (Refer to FIG. 11) which is sterilized and forms extraction pores (fine pores) for extracting body fluid on the subject's skin by contacting a fine needle 113a of the fine needle chip 110 with the subject's skin. The body fluid (tissue fluid) exudated from the extraction pore on the subject's skin which is thus formed by the puncture device 1 and the fine needle chip 110 is analyzed with a glucose concentration analysis device (Refer to FIGs. 22 and 33). By the analysis, a glucose concentration in the tissue fluid is calculated and a blood glucose level is predicted based on the value. Diabetes patients themselves monitor and manage a predicted blood glucose level. First, with reference to FIGs. 1 to 12, a configuration of the puncture device 1 according to an embodiment of the present invention is described in detail.

The puncture device 1 forms plural fine extraction pores which do not reach up to vascular plexus in a dermis although they penetrate a stratum corneum of epidermis of the skin and reach the stratum granulosum and the like of the epidermis, and exudates a tissue fluid from the extraction pores. This puncture device 1 comprises a main body 1a having a puncture mechanism for puncturing a subject's skin, and a timer unit 140 having a timer function described below. As shown in FIGs. 1 to 3, the main body 1a of the puncture device 1 comprises a rear cover 10, a front cover 20, a chip accommodation tool insertion member 30, an array chuck 40 being a piston section, a spring stopper 50, a release button 60, an ejector 70, a mainspring 80 (Refer to FIG. 3), and plural springs 90a to 90d (Refer to FIG. 3). Here, seven members (rear cover 10, front cover 20, chip accommodation tool insertion member 30, array chuck 40, spring stopper 50, release button 60, and ejector 70) except for the springs (mainspring 80 and plural springs 90a to 90d) are respectively made of synthetic resin. The puncture mechanism of the main body 1a of the puncture device 1 is principally configured by the array chuck 40, the spring stopper 50, the release button 60, and the mainspring 80.

[Configuration of respective elements of main body]

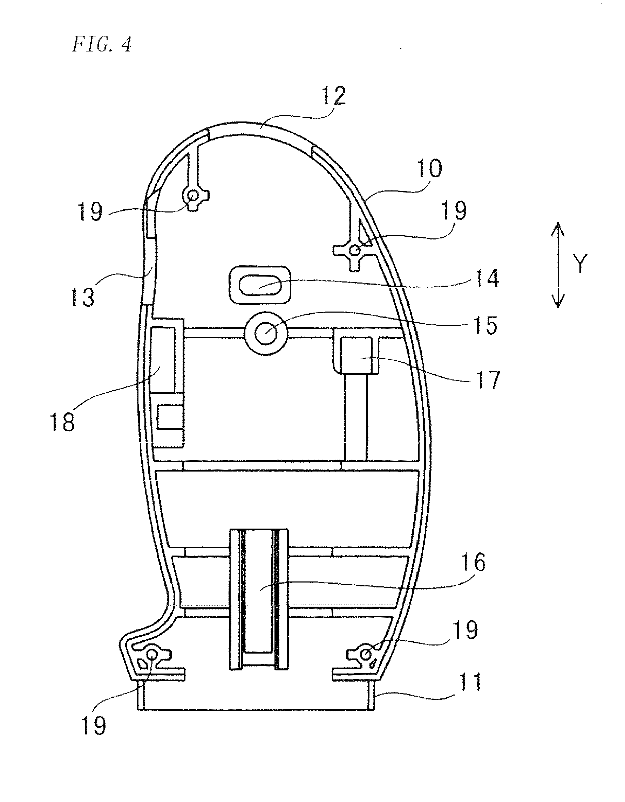

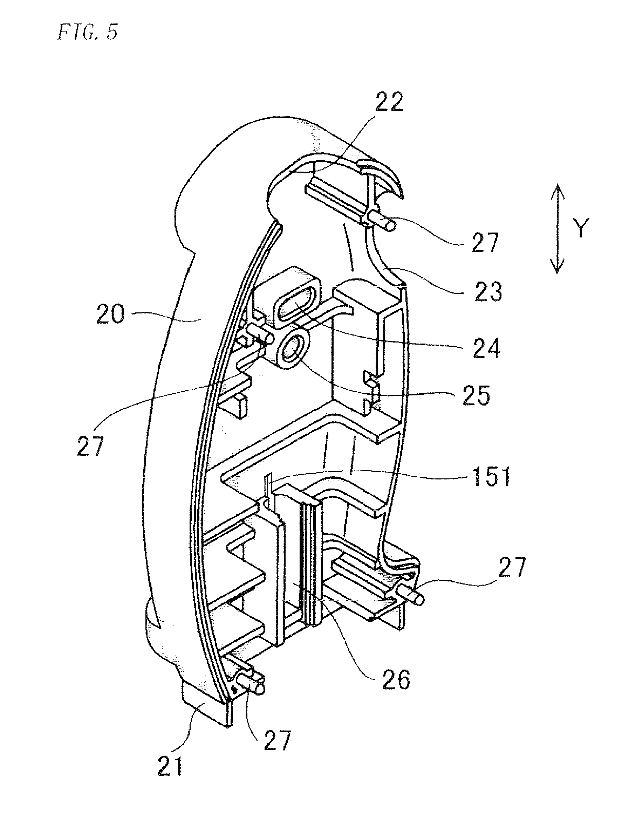

As shown in FIGs. 2 and 3, a housing consisting of the rear cover 10 and the front cover 20 is capable of accommodating therein the array chuck 40, the spring stopper 50, the release button 60, and the ejector 70, the mainspring 80, and the plural springs 90a to 90d. A fitting section 11 is formed on a lower part of the rear cover 10 for fitting the chip accommodation tool insertion member 30, as shown in FIGs. 3 and 4. Further, on an upper part of the rear cover 10, an opening 12 is formed for exposing a button section 72 of the ejector 70 so that the user can press. Further, on a side of the rear cover 10, an opening 13 is formed for exposing a button section 64 of the release button 60. Further, inside the rear cover 10, a concave 14 in which one end 52a of a spring contact section 52 of the spring stopper 50 is fit, a concave 15 in which a support shaft 63 of the release button 60 is engaged, a guide groove 16 for guiding a guide section 43 of the array chuck 40 which moves inside the housing in Y direction (vertical direction in FIGs. 1 to 5), spring installation sections 17 and 18 for installing the springs 90a and 90b respectively, and four pieces of boss insertion pores 19 in which four pieces of boss sections 27 of the front cover 20 are inserted (Refer to FIG. 5) are provided. Further, the spring 90c is installed in the guide groove 16.

As shown in FIGs. 3 and 5, and similarly to the rear cover 10, the front cover 20 comprise a fitting section 21 for fitting the chip accommodation tool insertion member 30, an opening 22 for exposing the button section 72 of the ejector 70 so that the user can press, an opening 23 for exposing the button section 64 of the release button 60, a concave 24 in which other end 52b of the spring contact section 52 of the spring stopper 50 is fitted, a concave 25 in which the support shaft 63 of the release button 60 is engaged, and a guide groove 26 for guiding the guide section 43 of the array chuck 40 moving inside the housing in Y direction. Further, the guide groove 26 (Refer to FIG. 5) is provided with the spring 90d (Refer to FIG. 3). Further, in the front cover 20, four pieces of the boss sections 27 are formed in a position opposite to four pieces of the boss insertion pores 19 of the rear cover 10 (Refer to FIG. 3). Therefore, four pieces of the boss sections 27 of the front cover 20 are inserted in four pieces of the boss insertion pores 19 of the rear cover 10, so that the front cover 20 is fit to the rear cover 10 in a positioning state.

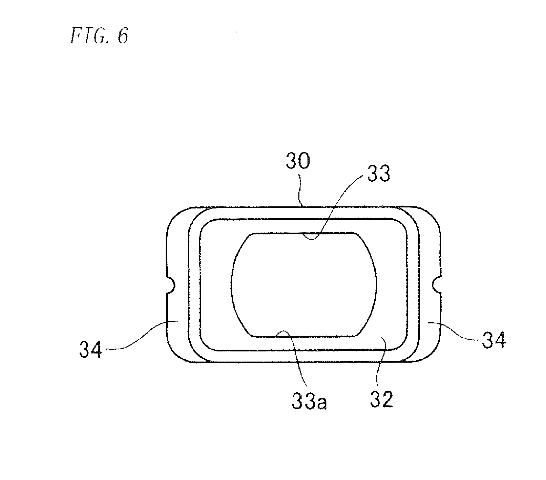

The chip accommodation tool insertion member 30 is provided for inserting a chip accommodation tool 120 accommodating the fine needle chip 110 (Refer to FIG. 11) when the fine needle chip 110 is mounted, and for inserting an empty chip accommodation tool 120 when the fine needle chip 110 which has been already used is disposed of. As shown in FIGs. 3 and 6, this chip accommodation tool insertion member 30 includes a fitting section 31 which is fitted on the fitting section 11 of the rear cover 10 and the fitting section 21 of the front cover 20 (Refer to FIG. 3), a contact surface 32 to be in contact with the skin of subject's arm, a through-hole 33 which has an opening 33a (Refer to FIG. 6) formed on the contact surface 32 and an opening 33b (Refer to FIG. 3) formed on the other side of the opening 33a, and two pieces of flange portions 34 formed so that they project outward from lateral outside surfaces.

Further, according to the present embodiment, the opening 33a formed on the contact surface 32 is configured so that the chip accommodation tool 120 for removably accommodating the fine needle chip 110 (Refer to FIG. 10) is insertable. Therefore, the chip accommodation tool 120 passing through the opening 33a can move through the through-hole 33 in Y direction.

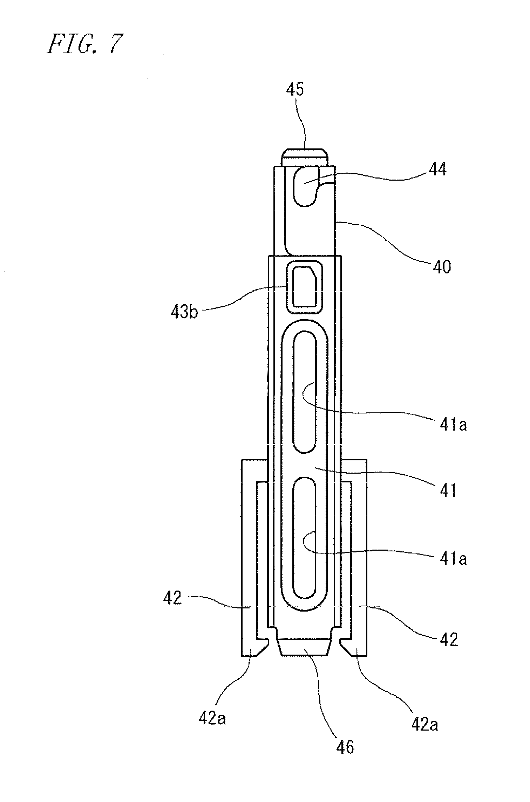

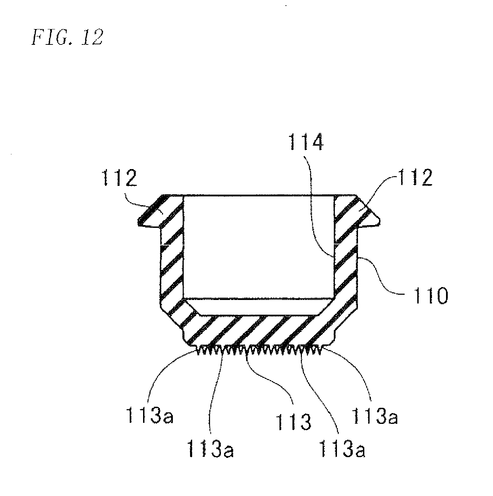

The array chuck 40 which functions as a piston for striking or contacting the fine needle chip 110 to or with the subject's skin is configured so that the array chuck 40 is capable of moving in Y direction along the guide groove 16 of the rear cover 10 and the guide groove 26 of the front cover 20. The fine needle chip 110 (Refer to FIG. 11) retained by the array chuck 40 is capable of moving in Y direction through the through-hole 33 of the chip accommodation tool insertion member 30. As shown in FIGs. 3 and 7, this array chuck 40 includes a body 41 which is provided with plural pores 41a for a purpose of lightweight, a pair of chuck sections 42 which are elastic and deformable and retain the fine needle chip 110 in engagement with a flange portion 112 (Refer to FIG. 12) of the fine needle chip 110, a guide section 43a inserted in the guide groove 16 of the rear cover 10 and a guide section 43b which is inserted in the guide groove 26 of the front cover 20 and has a tip end 43d projecting from a slit 151 described below, two pieces of engagement sections 44 in engagement with two pieces of lock sections 62 of the release button 60 described below, a convex 45 which has an insertion hole 45a (Refer to FIG. 3) capable of inserting a shaft section 51 of the spring stopper 50 described below, and a bush section 46 which is formed in the lower part of the body 41 (on a side of arrow mark Y1). Further, the tip end 42a in contact with the flange portion 112 of the fine needle chip 110 of the chuck section 42 is formed in a taper shape and formed in a hook shape so that the tip end 42a is capable of engaging with the flange portion 112. Further, the guide section 43a is formed so that the guide section 43a contacts with one end of the spring 90c arranged in the guide groove 16 of the rear cover 10, and the guide section 43b is formed so that the guide section 43b contacts with one end of the spring 90d arranged in the guide groove 26 of the front cover 20.

Here, according to the present embodiment, in a case where two pieces of engagement sections 44 are not engaged with two pieces of lock sections 62 of a release button 60 described below, the array chuck 40 is so configured that the fine needle chip 110 accommodated in the chip accommodation tool 120 is automatically retained by inserting the chip accommodation tool 120 (Refer to FIG. 10) in the opening 33a of the chip accommodation tool insertion member 30. Further, after retaining the fine needle chip 110, the array chuck 40 movable in Y direction is moved in a direction of arrow mark Y2 until the engagement section 44 is locked to the lock section 62.

Further, according to the present embodiment, in a case where two pieces of the engagement sections 44 are not engaged with two pieces of the lock sections 62 of the release button 60 described below, the fine needle chip 110 retained by the array chuck 40 is so configured that the fine needle chip 110 is automatically removed from the chuck section 42 of the array chuck 40 by inserting the chip accommodation tool 120 in the opening 33a of the chip accommodation tool insertion member 30.

Further, according to the present embodiment, the chuck section 42 is integrally formed with other sections (body 41, guide section 43a, 43b, engagement section 44, convex 45, and bush section 46) and all are made of synthetic resin.

The spring stopper 50 is provided for supporting the mainspring 80 which biases the array chuck 40 in a direction of arrow mark Y1. This spring stopper 50, as shown in FIG. 3, includes a shaft section 51 to be inserted in the mainspring 80 and a spring contact section 52 for preventing the mainspring 80 to be inserted in the shaft section 51 from escaping upward (in a direction of arrow mark Y2). Then, an end 52a on one side of the spring contact section 52 and an end 52b on other side thereof are formed so that they are respectively fitted in the concave 14 of the rear cover 10 and the concave 24 of the front cover 20 (Refer to FIG. 5).

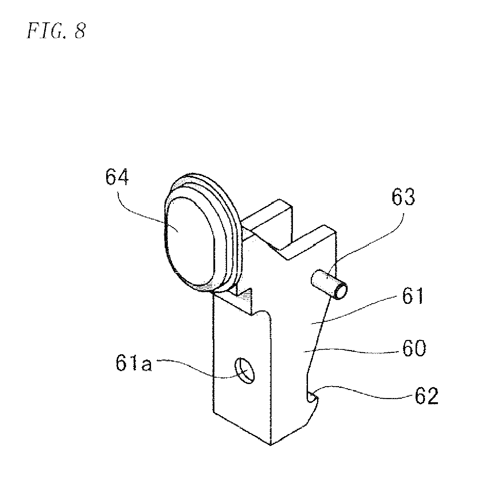

The release button 60, as shown in FIGs. 3 and 8, comprises a body 61, two pieces of the lock sections 62 which engage with two pieces of the engagement sections 44 of the array chuck 40, two pieces of the support shafts 63 which engage with the concave 15 of the rear cover 10 and the concave 25 of the front cover 20 (Refer to FTG. 5), and the button section 64 which is exposed from the opening 13 arranged in a side surface of the rear cover 10 and the opening 23 arranged in a side surface of the front cover 20 (Refer to FIG. 5). Further, a concave 61a to be in contact with one end of the spring 90b (Refer to FIG. 3) which is installed in the spring installation section 18 (Refer to FIGs. 3 and 4) of the rear cover 10 is formed in a side surface having the button section 64 of the body 61 thereon as shown in FIG. 8. Further, according to the present embodiment, two pieces of lock sections 62 have a function of locking the array chuck 40 which is moved in a direction of arrow mark Y2 against a bias force in a direction of arrow mark Y1 of a mainspring 80 described below.

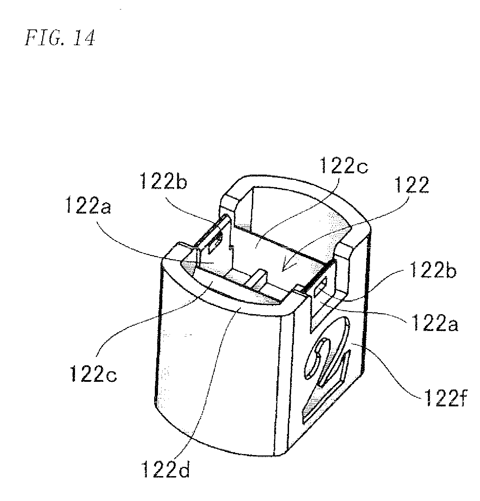

Further, according to the present embodiment, the ejector 70 has a function of discharging the chip accommodation tool 120 accommodating the fine needle chip 110 through the through-hole 33 (Refer to FIG. 3) of the chip accommodation tool insertion member 30. This ejector 70, as shown in FIG. 3, comprises a press sections 71 which presses an edge 121b (Refer to FIG. 10) and an edge 122d (Refer to FIG. 14) of the chip accommodation tool 120 which are described below, a button section 72 which is exposed from the opening 12 of the rear cover 10 and the opening 22 of the front cover 20 and can be pressed by the subject, and a contact section 73 in contact with one edge of the spring 90a which is installed in the spring installation section 17 of the rear cover 10. A boss section 73a which is inserted inside the spring 90a is formed in the contact section 73 so that it is possible to prevent release of the spring 90a from the spring installation section 17 of the rear cover 10.

The mainspring 80 is provided for biasing the array chuck 40 in a direction of arrow mark Y1. The shaft section 51 of the spring stopper 50 is inserted inside the mainspring 80 as shown in FIG. 3. Here, the one end 80a of the mainspring 80 is in contact with the spring contact section 52 of the spring stopper 50 and the other end 80b is in contact with upper surface of the engagement section 44 of the array chuck 40.

The spring 90a which is installed in the spring installation section 17 of the rear cover 10 and inserted in the boss section 73a of the contact section 73 of the ejector 70 has a function of biasing the ejector 70, which is pressed up in a direction of arrow mark Y2, in a direction of arrow mark Y1 as shown in FIG. 3. Further, the spring 90b which is arranged in the spring installation section 18 of the rear cover 10 and in the concave 61a (Refer to FIG. 8) of the release button 60 is provided for turning in a direction of arrow mark G1 the release button 60 which is turned around the support shaft 63 as a support point in a direction of arrow mark G2. Further, the springs 90c and 90d which are installed in the guide groove 16 of the rear cover 10 and the guide groove 26 (Refer to FIG. 5) of the front cover 20 have a function of pressing back in a direction of arrow mark Y2 the array chuck 40 which is moved in a direction of arrow mark Y1 due to a bias force of the mainspring 80. Therefore, it is possible to prevent the array chuck 40, which is moved in a direction of arrow mark Y1, from moving downward (in a direction of arrow mark Y1) from a specific position and to prevent the fine needle 113a of the fine needle chip 110 from puncturing deep in the subject's arm.

[Timer unit]

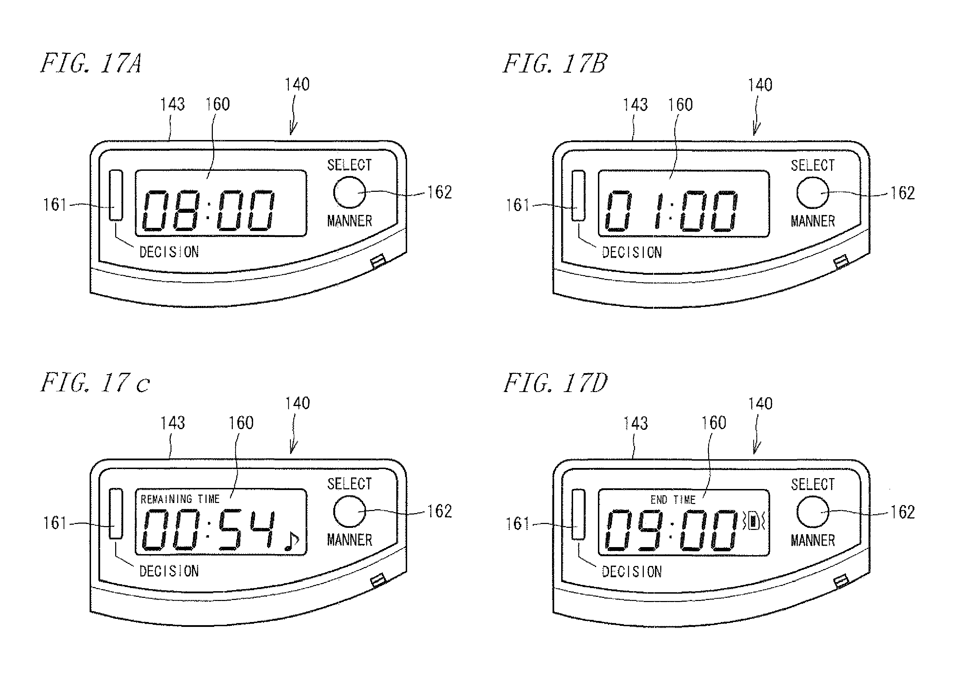

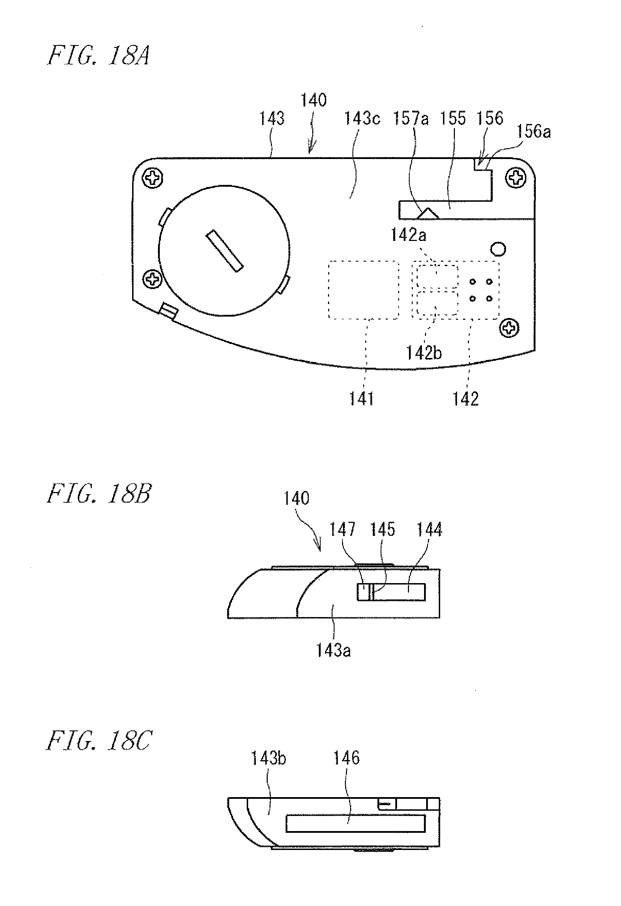

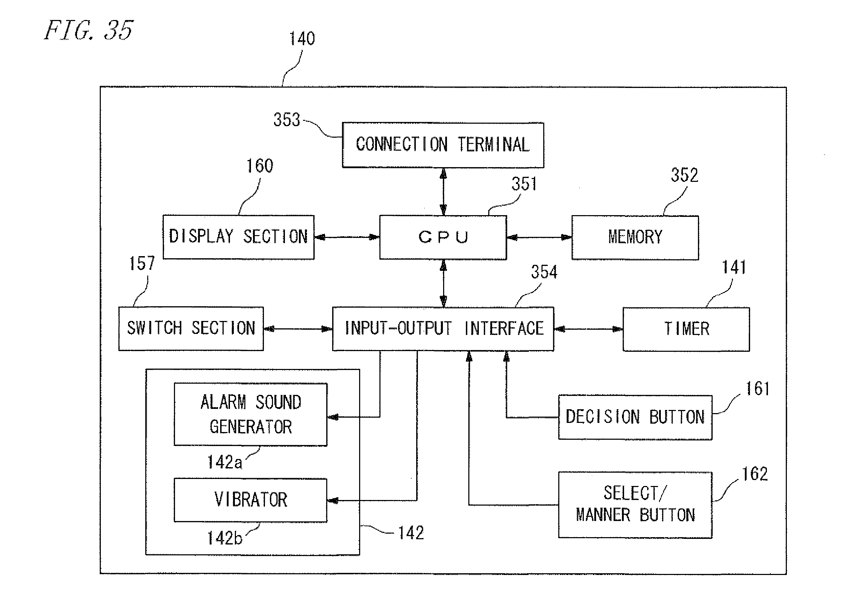

FIGs. 17A to 17D are explanatory plan views of a timer unit 140 in the puncture device 1 according to the present embodiment. FIG. 18A is an explanatory bottom view of the same, FIG. 18B is an explanatory upper view of the same, and FIG. 18C is an explanatory lower view of the same. Further, FIG. 35 is a block diagram of the timer unit 140. As shown in FIG. 35, the timer unit 140 comprises a timer 141, an alarm 142, a switch section 157, a display section 160, a decision button 161, a select/manner button 162, a CPU 351, a memory 352, a connection terminal 353, an input-output interface 354, and the like. The timer unit 140 is covered with a casing 143 made of synthetic resin. The timer 141 has a function of starting measurement of a specific time by a puncture action as described below. The alarm 142 has a function of notifying the subject that the specific time has passed. The CPU 351 is caused to control actions of respective types of components of the timer unit 140. According to the present embodiment, an alarm sound generator 142a emitting sound and a vibrator 142b emitting vibration are provided as the alarm 142, and at least one of them is caused to function by operation of the decision button 161 and the select/manner button 162.

Further, the user operates the decision button 161 and the select/manner button 162 for causing the CPU 351 to adjust time of the timer 141, set extraction time, and select a notifying method through the input-output interface 354. For example, according to the present embodiment, when the decision button 161 is pressed down in a state in which the timer unit 140 is not mounted on the main body 1a, a screen displays time as shown in FIG. 17A and a portion displaying "hour" blinks. When the select/manner button 162 is pressed down in this state, it is possible to change hour display. Further, when the decision button 161 is pressed down in a state that the portions of "hour" blinks, a portion displaying "minute" blinks. When the select/manner button 162 is pressed down in this state, it is possible to change minute display.

When the decision button 161 is pressed down in a state in which the portion displaying "minute" blinks, the screen displays extraction time as shown in FIG. 17B and the extraction time blinks. When the select/manner button 162 is pressed down in this state, it is possible to change the extraction time every 10 minutes. Further, when the decision button 161 is pressed down during blinking of the extraction time, it is possible to lock the extraction time and turn off a power supply of the display section 160. Thus, preparation for activating the timer is completed.

Next, when the array chuck 40 is ejectably loaded as described below, the power supply of the displays section 160 is turned on, and the extraction time set up by the user is displayed on the display section 160. Subsequently, when the array chuck 40 is ejected, "remaining time" is displayed on the display section 160 as shown in FIG. 17C. Further, when the select/manner button 162 is pressed down for short time in this state, it is possible to shift the display section 160 to a mode displaying "end time" as shown in FIG. 17D. Further, when the select/manner button 162 is pressed down for long time in a state in which "remaining time" or "end time" is displayed, it is possible to select a method of notifying the subject of end of the extraction time, by sound or vibration, or both of them. Then, which notifying method is selected is displayed by a symbol mark on the display section 160. FIG. 17C shows that sound is selected as a notifying method and FIG. 17D shows that vibration is selected as a notifying method. Further, when the decision button 161 or the select/ manner button 162 is pressed down in a state in which extraction time ends and the alarm 142 is activated, it is possible to stop the sound or the vibration which is emitted, or both of them.

The timer unit 140 is provided with the memory 352 which memorizes variety of information related to the subject and measurement. The memory 352 is composed of ROM and RAM. As the variety of information memorized by the memory 352, examples are a name of the subject (patient) and a lot and a type of gel being an extraction medium. The timer unit 140 is provided with the connection terminal 353 for transferring these types of information from the timer unit 140 to the measurement device or PC (personal computer). In a case where the puncture device of the present invention is utilized in a medical institution, the subject carries around the timer unit 140 with a gel reservoir member (collection member) for extraction being applied to the puncture site of the subject. A component subject to be measured in the extracted tissue fluid is measured, after the timer unit 140 and the gel reservoir member in which the tissue fluid is extracted and retained are collected after the specific extraction time has passed. Here, it is possible for a measurer to obtain information about the patient being the subject only by receiving the timer unit 140 and the gel reservoir member for extraction, so that work such as recording the variety of information by the measurer is not required.

Further, when extraction time and measurement date and time are memorized by the memory 352 of the time unit 140, an individual is not required to record separately, and therefore convenience improves.
Further, it is also possible to cause time when the subject has a meal to be memorized. It is possible to review it when the obtained data are analyzed by recording meal time when puncture is performed and measurement starts after the meal or history of meal time.

Further, it is possible to cause the timer unit 140 to record a past blood glucose level of the subject which is obtained by the self-monitoring of blood glucose (SMBG) and it is possible to consider it together with a result of AUC measurement which is currently obtained. Especially, in the case of the SMBG result which is measured in combination with the AUC measurement, the result is possible to be applied to AUC wave analysis.

It is also possible to display these outputs from the timer unit 140 on the display section 160 together with measurement time and it is possible to output the outputs to PC for data analysis from the connection terminal 353 for PC provided in the timer unit 140.

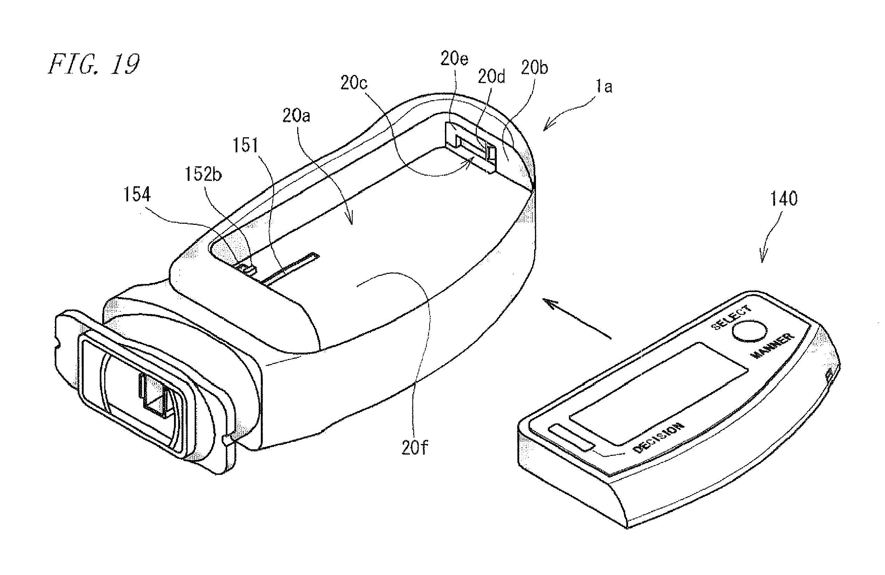

The timer unit 140 is removably mounted on the main body 1a as shown in FIG. 19. More particularly, a concave 20a having a shape and a size corresponding to outline of the timer unit 140 is formed in the front cover 20. When the timer unit 140 is mounted so that it fits inside the concave 20a of the front cover 20, obtained outline of the puncture device 1 is substantially continuous and even as shown in FIG. 1. As shown in FIG. 19, an opening 20c is formed in an upper side wall 20b which defines the concave 20a of the front cover 20. An engagement piece 20e is arranged inside the opening 20c, and an engagement nail 20d projecting outward from the opening 20c is arranged on the tip end of the engagement piece 20e. This engagement piece 20e is a cantilever beam with an end on the side of the engagement nail 20d being a free end and the engagement piece 20e is swingable within a specific range with a root part thereof as a basic point.

When the timer unit 140 is mounted on the main body 1a, as shown in FIG. 18B, a guide groove 144 for guiding the engagement nail 20d of the engagement piece 20e is formed in one side surface 143a (side surface located upper side in use of the puncture device 1 with the timer unit 140 being mounted on the main body 1a) of a casing 143. A convex line 145 perpendicular to the longitudinal direction of the guide groove 144 is formed in depth of the guide groove 144 (left side in FIG. 18B).

Further, a guide groove 146 for guiding a rib (not shown) which is formed in a lower side wall defining the concave 20a is formed in other side surface 143b which faces the one side surface 143a of the casing 143.

In a case where the timer unit 140 having the above-mentioned configuration is mounted on the main body 1a so that the timer unit 140 is positioned inside the concave 20a of the front cover 20, the engagement nail 20d of the engagement piece 20e moves in the guide groove 144 in the one side surface 143a of the casing 143, and the rib formed in the lower side wall moves in the guide groove 146 in the other side surface 143b of the casing 143. Here, the engagement nail 20d of the engagement piece 20e moves in the guide groove 144 in contact with the bottom surface 144a of the guide groove 144, and passes over the convex line 145, and is engaged with an engagement concave 147. Mount of the timer unit 140 on the main body 1a is completed by engagement between the engagement nail 20d and the engagement concave 147, and it prevents the timer unit 140 from being removed from the main body 1a due to contact and the like.

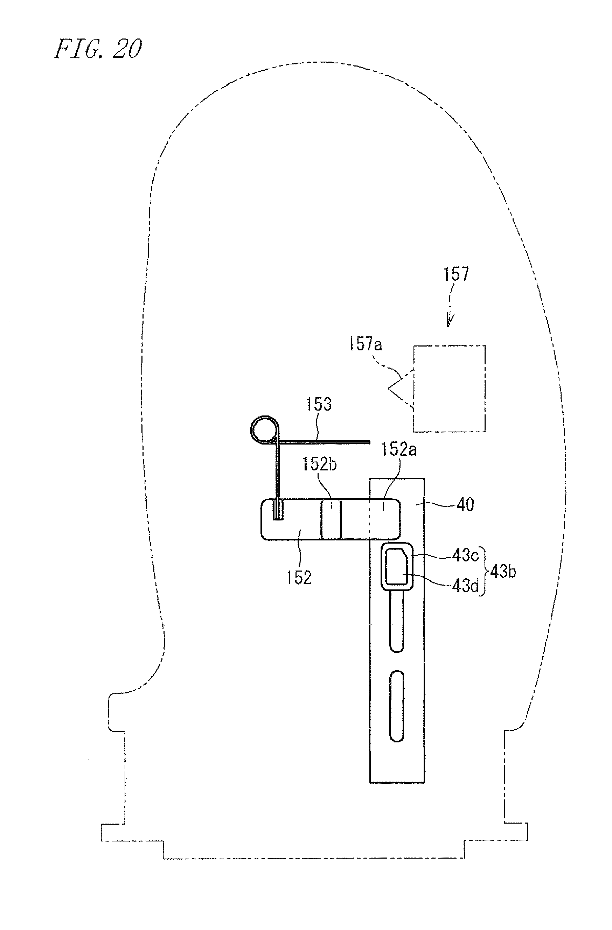

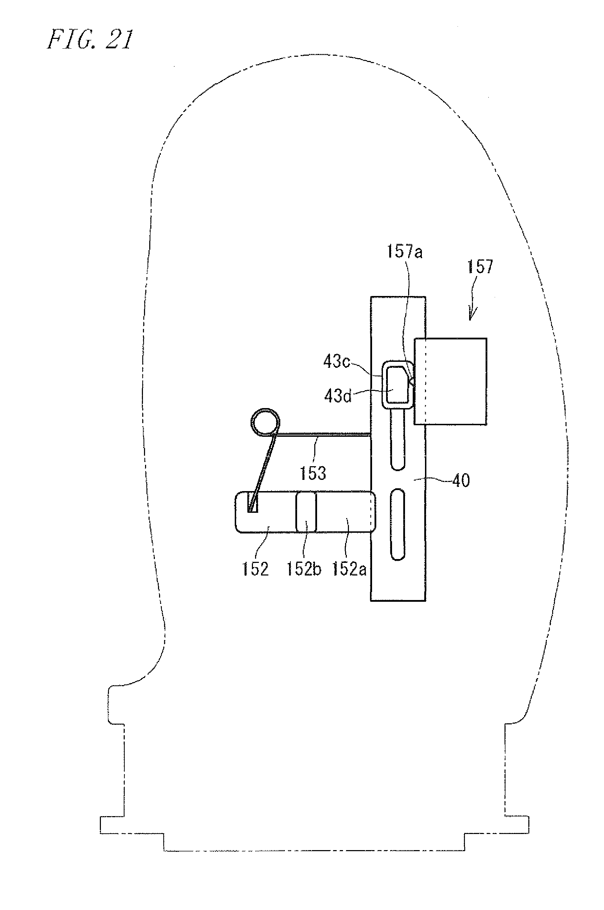

FIG. 20 is an explanatory view showing a state before the fine needle chip is mounted on the array chuck 40. FIG. 21 is an explanatory view showing a state in which the array chuck 40 mounted with the fine needle chip is moved to an ejectable position. FIGs. 20 and 21 show arrangement of the array chuck 40 and the like in the puncture device 1, which is viewed from the side of the timer unit 140, that is, the side of the front cover 20. Here, illustration of the fine needle chip is omitted in FIGs. 20 and 21 for easy understanding. Further, because the timer unit 140 is not mounted on the main body 1a in the state shown in FIG. 20, the switch section 157 in the timer unit 140 is drawn by an imaginary line. As shown in FIGs. 20 and 21, the guide section 43b is protectively provided in the upper end of one side surface (facing the timer unit 140 mounted on the main body 1a) of the array chuck 40 being a piston section. This guide section 43b comprises a basic section 43c locked to the one side surface and a tip end 43d (first projection) which is integrally formed with the basic section 43c and thinner than the basic section 43c. The tip end 43d of the guide section 43b projects outward from the slit 151 which is formed in a bottom wall 20f defining the concave 20a of the front cover 20 (Refer to FIGs. 3 and 19).

Further, an engagement piece 152 which is movable is arranged in the housing configured by the front cover 20 and the rear cover 10. This engagement piece 152 has a second projection 152a and a third projection 152b which projects in a perpendicular direction to a projection direction of the second projection 152a. The engagement piece 152 is biased by a coil spring 153 being a bias means which is arranged in the housing in such a direction that the engagement piece 152 is engaged with the tip end 43d of the guide section 43b. The third projection 152b of the engagement piece 152 projects outward from a slit 154 (Refer to FIGs. 3 and 19) formed in the bottom wall 20f, similarly to the tip end 43d of the guide section 43b.

In a state shown in FIG. 20, the timer unit 140 is not mounted on the main body 1a. In this state, the engagement piece 152 proceeds due to a bias force of the coil spring 153 in such direction that the engagement piece 152 engages with the tip end 43d of the guide section 43b. Therefore, even though the array chuck 40 mounted with the fine needle chip 110 is forced to push into the device, it is impossible to push into because the tip end 43d of the guide section 43b contacts with the second projection 152a of the engagement piece 152.

On the other hand, when the timer unit 140 is mounted on the main body 1a as described below, it is possible to mount the fine needle chip 110 on the array chuck 40 and push the array chuck 40 into the device since the engagement piece 152 moves in such a direction that the engagement with the tip end 43d of the guide section 43b is released. As shown in FIG. 21, when the array chuck 40 is pushed into the device, the tip end 43d of the guide section 43b presses the tip end 157a of the switch section 157.

A guide groove 155 being a groove is formed in a position which is on a rear surface or the bottom surface 143c (Refer to FIG. 18A) of the casing 143 of the timer unit 140 and which faces the slit 151 when the timer unit 140 is mounted on the main body 1a. The switch section 157 with the tip end 157a projecting into the guide groove 155 is provided inside the casing 143. The tip end 157a of the switch section 157 is configured so that it can recede from the guide groove 155 by pressure.

Further, a notch 156 (Refer to FIG. 18A) is formed in a side surface which is a side surface of the casing 143 and where the casing 143 is mounted on the main body 1a. The notch 156 is formed in such a position that the bottom surface 156a thereof contacts with the third projection 152b of the engagement piece 152 when the timer unit 140 is mounted on the main body 1a.

[Lock mechanism and turn-on mechanism]

Next, a lock mechanism which inhibits a puncture action while the timer unit is not mounted and a turn-on mechanism which turns on a power supply of the display section 160 by loading the fine needle chip 110 on the array chuck 40 are explained.

In a state in which the timer unit 140 is not mounted on the main body 1a as shown in FIGs. 19 and 20, the array chuck 40 is biased in a direction of puncture by the mainspring 80 as shown in FIG. 3, and the tip end (the first projection, the press member) 43d of the guide section 43b of the array chuck 40 projects outward from the slit 151. Further, the engagement piece 152 is biased by the coil spring 153 in such direction that the second projection 152a thereof engages with the tip end 43d being the first projection. The second projection 152a of the engagement piece 152 is located on an upper side of the tip end 43d, that is, in depth side or inner side (in Y2 direction) of the array chuck 40 with the tip end 43d being as a basis. Therefore, in this state, it is impossible to push the array chuck 40 up to a position that the fine needle chip 110 is mounted on the array chuck 40 and the fine needle chip 110 can be ejected.

When the timer unit 140 is mounted on the main body 1a, the bottom surface 156a of the notch 156 which is formed in a side wall of the casing of the timer unit 140 contacts with the third projection 152b of the engagement piece 152, and moves the engagement piece 152 in such a direction that the second projection 152a recedes from the tip end 43d against bias force of the coil spring 153. Therefore, since engagement between the tip end 43d of the array chuck 40 and the second projection 152a of the engagement piece 152 is released (lock released), it is possible that the array chuck 40 moves opposite to a puncture direction.

When the fine needle chip 110 is mounted on the array chuck 40 and the array chuck 40 is pushed into the device opposite to a puncture direction after the timer unit 140 is mounted on the main body 1a, the tip end 43d as a press member moves in the guide groove 155 of the casing 143 of the timer unit 140, and the tip end 43d presses the tip end 157a of the switch section 157, and the tip end 43d retreats the tip end 157a from inside of the guide groove 155. According to the present embodiment, the power supply of the display section 160 is turned on by pressure of the switch section 157 by the tip end 43d as a press member, and the extraction time set up by the user is displayed on the display section 160. More particularly, when the CPU 351 recognizes the pressure of the switch section 157 through the input-output interface 354, the CPU 351 can turn on the power supply of the display section 160. Here, the power supply of the display section 160 is turned off after a given time has passed.

Next, when the array chuck 40 is ejected by pressing the button section 64 of the release button 60, engagement between the tip end 43d of the array chuck 40 and the tip end 157a of the switch section 157 is released, and the tip end 157a of the switch section 157 again projects inside the guide groove 155. According to the present embodiment, the CPU 351 recognizes release of pressure of the tip end 157a or a puncture action through the input-output interface 354 and causes the timer 141 to start time measurement.

[Chip accommodation kit]

Next, with reference to FIGs. 1, 3, 7 and 9 to 16, a chip accommodation kit 100 composed of a fine needle chip 110 which is mounted on the array chuck 40 of the puncture device 1 according to the present embodiment, a chip accommodation tool 120 accommodating the fine needle chip 110, and a sterilization preservation seal 130 are explained in detail.

The fine needle chip 110 is mounted in the array chuck 40 (Refer to FIG. 7) of the above-mentioned puncture device 1 (Refer to FIG. 1) for use and has plural fine needles 113a for forming plural extraction pores to exudate a tissue fluid (body fluid) from the subject's skin. The fine needle chip 110 is formed in a shape of substantial rectangle in a plane view, as shown in FIGs. 10 to 12. The fine needle chip 110 includes a pair of projections 111 which are arranged so as to project outward from lateral outside surfaces, a pair of flange portions 112 which are arranged so as to project outward from longitudinal outside surfaces, a fine needle array section 113 which has 305 pieces of fine needles 113a, and a concave 114 in which the bush section 46 (Refer to FIG. 7) of the array chuck 40 of the puncture device 1 described above is inserted. Further, a pair of projections 111 are formed so that they are engaged by an engagement pore 122b of the chip accommodation tool 120 described later. A pair of flange portions 112 are formed so that they engage the tip end 42a of the chuck section 42 (Refer to FIG. 7) of the array chuck 40. Here, the fine needle chip 110 together with 305 pieces of fine needles 213a are formed of synthetic resin. Now, except for the fine needle chip 110 including the fine needle array section 113 having 305 pieces of the fine needles 113a described above, other fine needle chips such as a fine needle chip including an fine needle array section having 189 pieces of fine needles may be used.

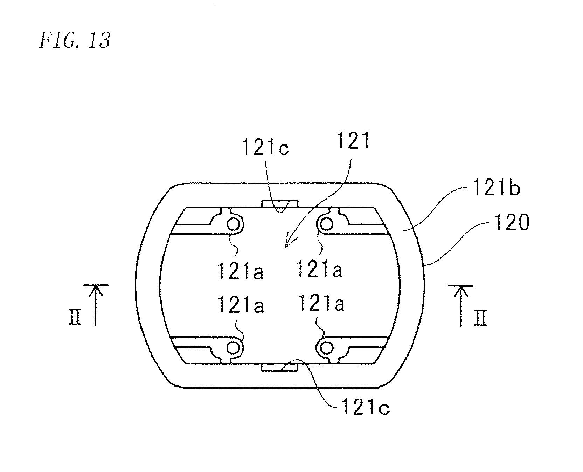

According to the present embodiment, the chip accommodation tool 120 formed of synthetic resin includes an opening 121 for accommodating the fine needle chip 110 (Refer to FIG. 10) before use which is sterilized and an opening 122 for accommodating the fine needle chirp 110 after use which is punctured on the subject's skin, as shown in FIGs. 10 and 13 to 16. The opening 121 and the opening 122 are arranged in an opposite side to each other. To the opening 121, the sterilization preservation seal 130 described below is applied for sealing the opening 121 which accommodates the fine needle chip 110 unused. Further, as shown in FIGs. 10 and 13, the opening 121 has four pieces of support sections 121a which support side surfaces of the fine needle chip 110 before use which is sterilized, an edge 121b which contacts with the press section 71 (Refer to FIG. 3) of the ejector 70, and an allowance 121c which is formed so that the projection 111 (Refer to FIG. 10 and 11) of the fine needle chip 110 retained by the support section 121a does not contact with the edge 121b.

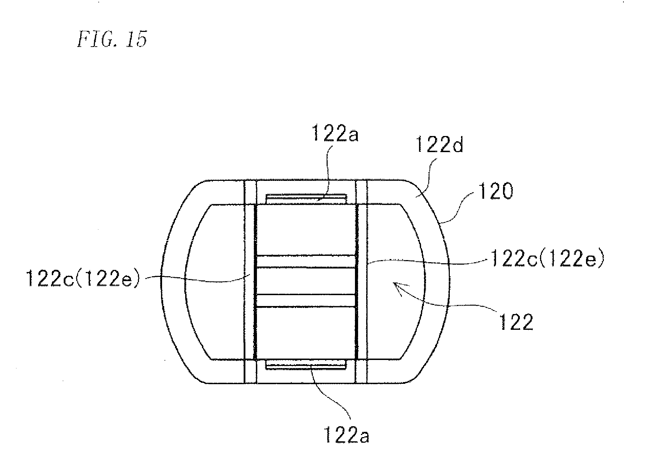

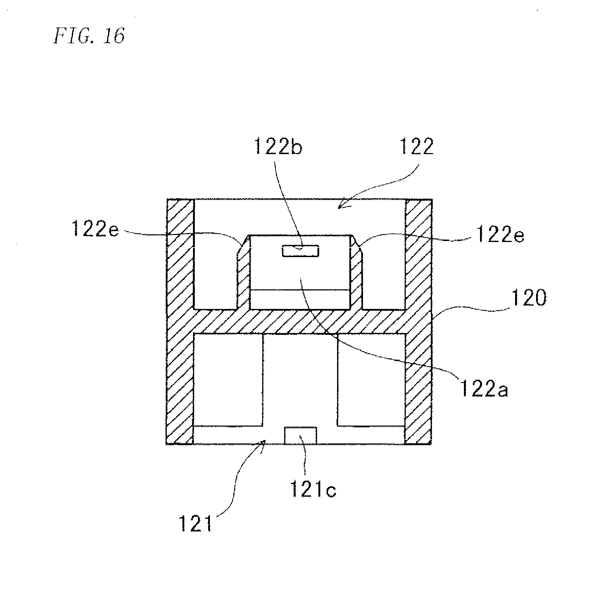

Further, according to the present embodiment, as shown in FIGs. 14 and 15, the opening 122 includes the retention section 122a which has the engagement pore 122b where the projection 111 (Refer to FIGs. 10 and 11) of the fine needle chip 110 which is already used and punctured on the subject's skin is inserted. Further, the opening 122 includes a release piece 122c which releases engagement between the chuck section 42 (Refer to FIG. 7) of the array chuck 40 of the puncture tool 1 and the flange portion 112 of the fine needle chip 110, and the edge 122d which contacts with the press section 71 (Refer to FIG. 3) of the ejector 70. A tip end 122e of the release piece 122c is formed in a taper shape as shown in FIG. 16. Further, a mark "2" is engraved on the side surface 122f of the chip accommodation tool 120 for easy confirmation in a case where the opening 122 is arranged upside.

The sterilization preservation seal 130 is formed of aluminum film and has a function of inhibiting adhesion of viruses, germs, and the like to the fine needle chip 110 which is sterilized by Y-ray irradiation. The sterilization preservation seal 130 is applied so as to cover the opening 121 which accommodates the fine needle chip 110 before use, as shown in FIGs. 9 and 10. Further, the sterilization preservation seal 130 is applied so as to cover "2" engraved on the side surface 122f of the chip accommodation tool 120 as described above. On a portion applied to the side surface 122f of the chip accommodation tool 120, a mark "1" is engraved for easy confirmation in a case where the opening 121 is arranged upside as shown in FIG. 9.

According to the present embodiment, there is provided the array chuck 40 for retaining the fine needle chip 110 by inserting the chip accommodation tool 120 in the opening 33a of the chip accommodation tool insertion member 30, in a case where engagement between the engagement section 44 of the array chuck 40 and the lock section 62 of the release button 60 is released. Therefore, it is possible that the subject causes the chuck section 42 of the array chuck 40 to retain the flange portion 112 of the fine needle chip 110 only by moving the puncture tool 1 in such way that the chip accommodation tool 120 is inserted in the opening 33a of the chip accommodation tool insertion member 30. Here, because the lock section 62 (release button 60) which locks the array chuck 40 by engaging with the engagement section 44 of the array chuck 40 is provided and the array chuck 40 is configured so that it can move in Y direction, it is possible that the fine needle chip 110 is retained in the array chuck 40 and the array chuck 40 is locked by the lock section 62 in a state in which the array chuck 40 is moved in a direction of arrow mark Y1 against a bias force by the mainspring 80. Therefore, the subject can set the puncture device 1 to a lock state in which the array chuck 40 retaining the fine needle chip 110 is biased in a direction toward the subject's skin (direction of arrow mark Y2). Thus, the subject can set to a state in which the puncture device 1 can form the fine pores on the subject's skin only by moving the puncture device 1 without requiring troublesome work. Because, by pressing the button section 64 of the release button 60 from this state, engagement between the engagement section 44 of the array chuck 40 and the lock section 62 is released, the fine needle chip 110 can pass through the opening 33a of the chip accommodation tool insertion member 30 and move toward a direction of arrow mark Y2, and the fine pores can be formed at the puncture site of the subject's skin.

Further, according to the present embodiment, when the chip accommodation tool 120 which is empty and does not accommodate the fine needle chip 110 is inserted in the opening 33a of the chip accommodation tool insertion member 30 in a case where the fine needle chip 110 is retained by the array chuck 40 and the engagement between the engagement section 44 of the array chuck 40 and the lock section 62 is released, it is possible that the subject easily removes the fine needle chip 110 which is already used and retained by the array chuck 40 in a state of engagement release from the lock section 62 only by moving the puncture device so as to insert the chip accommodation tool 120 in the opening 33a of the chip accommodation tool insertion member 30. Therefore, it is possible that the subject safely disposes of the used fine needle chip 110 without touching the used fine needle chip 110.

[Example of AUC measurement]

The puncture device according to the present invention can be preferably used for measuring blood glucose AUC. Hereinafter, an example of a blood glucose AUC measurement using the puncture device of the present invention is described.

First, before explanation of the example, significance and others of the blood glucose AUC measurement used in the present invention are explained.

[AUC measurement]

Conventionally, as a method of obtaining a value which reflects a total quantity of component circulating in vivo within a specific period, there has been known a method of measuring an area under the blood concentration time curve (AUC). AUC refers to an area which is enclosed with a horizontal axis (time axis) and a curve (in vivo component blood concentration-time curve) described by a graph representing time lapse of a specific blood concentration of in vivo component. For example, in a case of oral drug administration, it is difficult to directly determine a quantity of the drug taken up in the circulating blood. However, it is possible to predict a total quantity of the drug taken up in the circulating blood by using a value obtained from AUC measurement.

In the AUC measurement, blood is sampled plural times every specific time, a specific component quantity in the blood which is sampled at respective time points is acquired, and a graph which represents time lapse of the blood concentration of the in vivo component (specific blood concentration of in vivo component) is obtained.

Further, the blood glucose AUC is an area (unit: mg·h/dl) which is enclosed with curve described by a graph representing time lapse of a blood glucose level and a horizontal axis. The blood glucose AUC is an index which is used for response evaluation of insulin and oral drugs in diabetes treatment. For example, it is possible to predict a total quantity of the glucose circulating in vivo of a subject after glucose load, by measuring based on the blood glucose AUC a value which reflects a total quantity of the glucose (blood sugar) circulating in the blood within a specific time after the glucose load (after meal).

As significance of such measurement of blood glucose AUC, it is considered that the blood glucose AUC measurement can control influence caused by personally different glucose metabolism. In other word, because there is a personal difference in time for showing reaction in the blood glucose level to the glucose load, it is impossible to recognize whether the blood glucose level is at a rise time or at a peak time, by measuring only the blood glucose level at a certain time after the glucose load. Further, even if the blood glucose level at the peak time can be measured, it is still impossible to recognize how long such high blood glucose state lasts. In this respect, in a case where the blood glucose AUC is measured, since a value which reflects a total quantity of the blood glucose circulating in the blood within specific period can be obtained, the measurement value is not influenced by time until the blood glucose level shows reaction to the glucose load. Further, it is possible to predict how long the high blood glucose state lasts based on the measurement value. Therefore, by measuring the blood glucose AUC, it is possible to obtain a value useful for predicting the glucose tolerance due to the glucose load without influence of personally different glucose metabolism.

In the blood glucose AUC measurement, a blood glucose AUC is ordinarily obtained by sampling blood every specific time (e.g. every 30 minutes), obtaining a blood glucose level of the sampled blood respectively, obtaining a graph representing time lapse of the blood glucose level, and obtaining an area enclosed with a curve described by the graph and a horizontal axis.

However, as described above, the conventional AUC measurement method has problems of plural times of blood samplings required within a specific period, high invasiveness, and a heavy burden on the subject.

Therefore, in PCT/JP2009/63668, the present applicant proposes an in vivo component measurement method capable of obtaining a value which reflects a total quantity of components to be measured which circulates in biological body within a specific period while decreasing a subject's burden. According to this in vivo component measurement method, an extraction medium made of pure water is used for extracting the tissue fluid from biological body and accumulating the tissue fluid.

Meanwhile it is considered that the quantity of thus extracted component to be measured, specifically the glucose extraction quantity changes in response to skin states which differ among the subjects. In this consideration, it is possible to calculate a blood glucose level more accurately by amending the glucose extraction quantity with glucose permeability (P) (indicating easiness for glucose to be extracted). For example, according to the in vivo component measurement method of the above-mentioned PCT/JP2009/63668, skin of the subject is punctured with a fine needle and the tissue fluid is extracted through the skin with a fine pore formed thereon. However, even if skins are punctured with the same fine needles, a quantity of extracted glucose of a subject who has soft and thin stratum corneum is large because the fine pore is easily formed. On the other hand, a quantity of the extracted glucose of a subject who has hard and thick stratum corneum is small because the fine pore is hard to be formed. Therefore, U.S. Patent Publication No. 2007/0232875 proposes that glucose permeability (P) in the extracted site of the subject is predicted and a blood glucose level is calculated with computation formula (BG = J/P where BG represents calculated blood glucose level and J represents extracted glucose quantity).

Prediction principle of the glucose permeability (P) in a measurement method described in Patent Publication No. 2007/0232875 is described below. It is known that electrolyte concentration in the tissue fluid is substantially similar among plural subjects having different blood glucose levels. For this reason, it is possible to predict a degree of tissue fluid permeating the skin (i.e. glucose permeability (P)) by measuring the electrolyte quantity which is included in the tissue fluid extracted through the skin. Therefore, pure water containing no electrolyte is used as an extraction medium holding the extracted tissue fluid, electricity is supplied to the extraction medium with the tissue fluid extracted, and electric conductivity (K) is measured so that the electrolyte quantity included in the extracted tissue fluid can be predicted. In other words, it is possible to predict the glucose permeability (P) from the electric conductivity (K) of the electrolyte of the extraction medium with the tissue fluid extracted.

According to the in vivo component measurement method related to PCT/JP2009/63668 using the glucose permeability prediction method described in U.S. Patent Publication No. 2007/0232875, it is possible to measure (predict) a blood glucose AUC of the subject at certain accuracy without giving burden on the subject. However, based on knowledge that quantity of sodium ion among various species of ions ($Na^+$, $K^+$, $Ca^{2+}$, $Mg^{2+}$, etc.) is much more highly correlated with the glucose extraction quantity than that of the other ions, glucose permeability is obtained based on only quantity of sodium ion extracted in the example described below.

[Example 1 of Blood glucose AUC measurement]

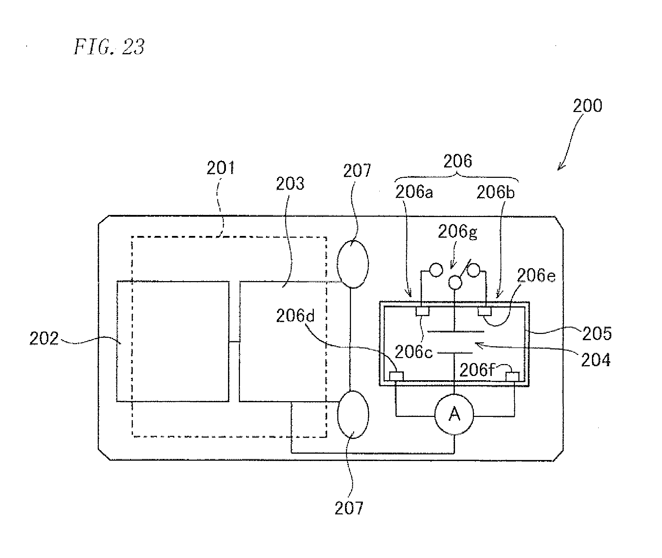

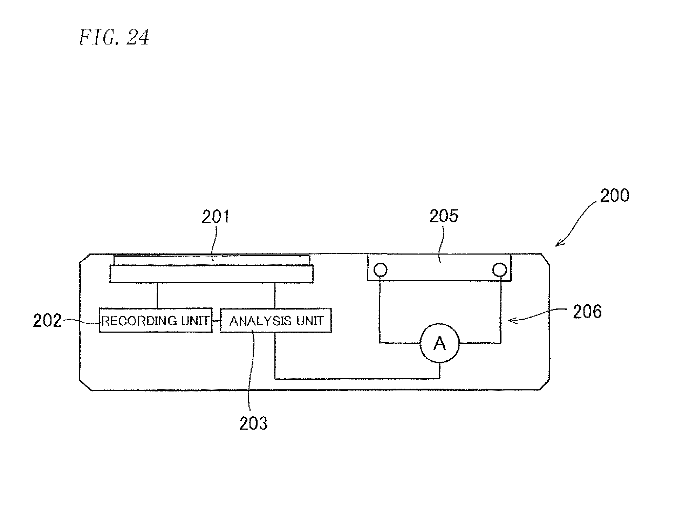

As shown in FIGs. 22 to 24, a measurement device 200 used in Example 1 comprises a display unit 201, a recording unit 202, an analysis unit 203, a power supply 204, an installation unit 205 being a setting unit for installing a sensor chip 300 and a collection member 400, an electric circuit 206 connected to the sensor chip 300 installed in the installation unit 205, and an operation button 207 for a user (subject) to operate the measurement device 200.

The display unit 201 has a function of displaying measurement result by the analysis unit 203 and data recorded in the recording unit 202. The recording unit 202 is provided for storing past data. The analysis unit 203 has a function of calculating a glucose concentration and a concentration of electrolyte (sodium ion) based on an output value of the electric circuit 206. The installation unit 205 has a concave shape and is configured so as to install the sensor chip 300 and the collection member 400. The electric circuit 206 includes a glucose concentration measurement circuit 206a and a sodium ion concentration measurement circuit 206b. The glucose concentration measurement circuit 206a includes terminals 206c and 206d which are exposed inside the installation unit 205. The sodium ion concentration measurement circuit 206b includes terminals 206e and 206f which are exposed inside the installation unit 205. The electric circuit 206 includes a switch 206g for switching the glucose concentration measurement circuit 206a and the sodium ion concentration measurement circuit 206b. The user can switch the glucose concentration measurement circuit 206a and the sodium ion concentration measurement circuit 206b by operating the operation button 207 to operate the switch 206g. The operation button 207 is provided for switching the switch 206g and switching display of the display unit 201.

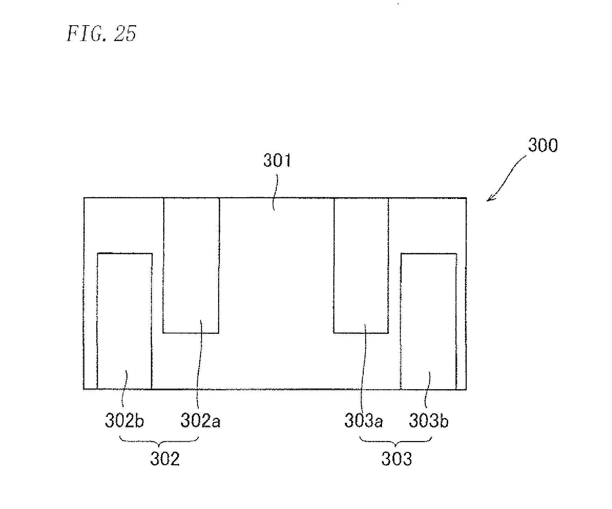

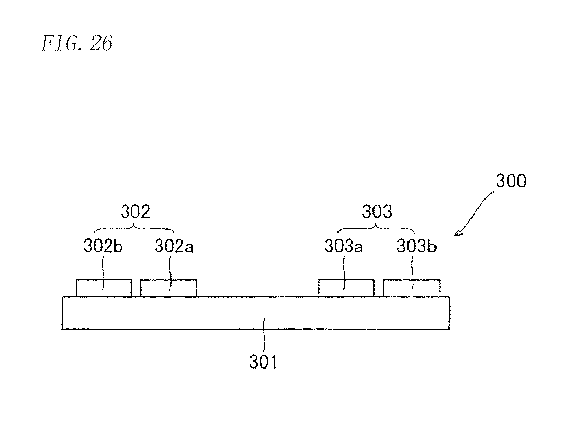

As shown in FIGs. 25 and 26, the sensor chip 300 comprises a substrate 301 made of synthetic resin, a pair of glucose concentration measurement electrodes 302 which are arranged on an upper surface of the substrate 301, and a pair of sodium ion concentration measurement electrodes 303 which are arranged on the upper surface of the substrate 301. The glucose concentration measurement electrode 302 consists of a work electrode 302a with a GOD enzyme membrane (GOD: glucose oxidase) formed on a platinum electrode and a counter electrode 302b formed of a platinum electrode. On the other hand, the sodium ion concentration measurement electrode 303 consists of a sodium ion selective electrode 303a which is made of silver/silver chloride and has a sodium ion selection membrane and a silver/silver chloride electrode 303b being a counter electrode. The work electrode 302a and the counter electrode 302b of the glucose concentration measurement electrode 302 respectively contact with the terminals 206c and 206d of the glucose concentration measurement circuit 206a, in a state in which the sensor chip 300 is installed in the installation unit 205 of the measurement device 200. Similarly, the sodium ion selective electrode 303a of the sodium ion concentration measurement electrode 303 and the silver/silver chloride electrode 303b respectively contact with the terminals 206e and 206f of the sodium ion concentration measurement circuit 206b, in a state in which the sensor chip 300 is installed in the installation unit 205 of the measurement device 200.

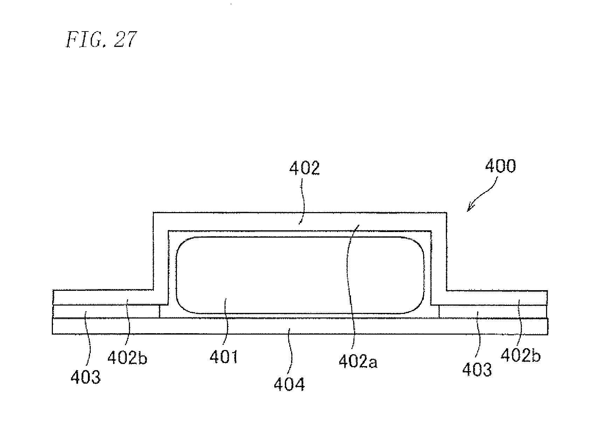

As shown in FIG. 27, the collection member 400 has such a configuration that a gel 401 having moisture (substantially containing no $Na^+$) capable of retaining the tissue fluid extracted from the subject's skin is supported by a support member 402. The gel 401 in Example 1 is made of polyvinyl alcohol and contains pure water as an extraction medium.

The support member 402 has a support main body 402a having a concave portion and a flange portion 402b formed in outer periphery of the support main body 402a, and the gel 401 is retained inside the concave portion of the support main body 402a. An adhesive layer 403 is formed on a surface of the flange portion 402b, and a peel-off paper 404 for sealing the gel 401 retained in the concave portion is applied to the adhesive layer 403 in a premeasurement state. During measurement, the gel 401 and the adhesive layer 403 are exposed by removing the peel-off paper 404 from the adhesive layer 403 and the collection member 400 is enabled to be applied and fixed to the subject's skin through the adhesive layer 403 in a state in which the gel 401 contacts to the subject's skin.

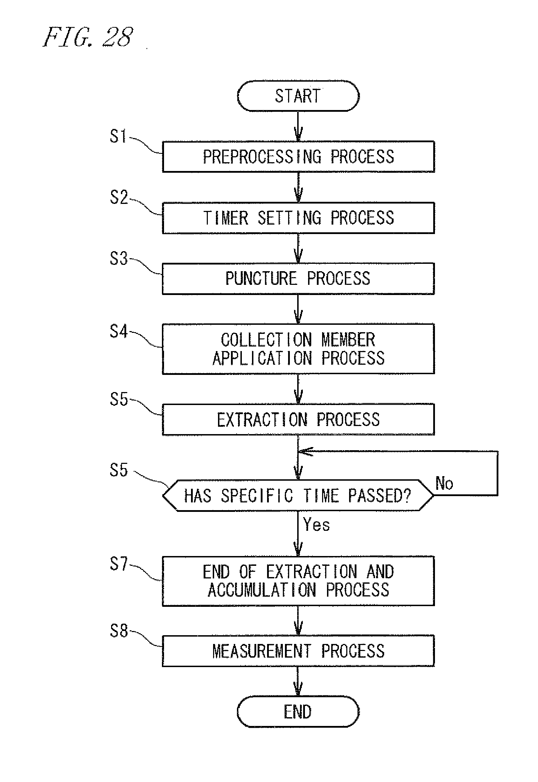

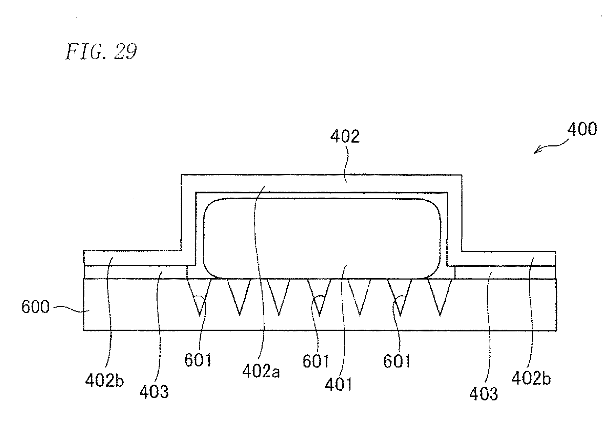

FIG. 28 is a flowchart showing a measurement procedure of the blood glucose AUC measurement method according to Example 1. FIGs. 29 to 31 are explanatory views of the measurement procedure of this measurement method. First, with reference to FIG. 28, outline of the measurement procedure of the blood glucose AUC measurement method according to Example 1 is explained. Among steps shown in FIG. 28, Steps S1 to S7 are carried out by those practicing measurement, and Step S8 is carried out by the measurement device 200.

First, a site to be measured of the subject is cleaned (Step S1). Next, tissue-fluid extraction time is set up using the timer unit 140 (at this stage, the timer unit 140 is not mounted on the main body 1a), and time is set up as needed in a case where end time is displayed by the display section 160 of the timer unit 140 (Step S2). Subsequently, the timer unit 140 is mounted on the main body 1a, the array chuck 40 is ejected, and the puncture action is carried out (Step S3). Next, the collection member 400 is fit at measurement site (Step S4), tissue fluid extraction starts and accumulation of glucose and sodium ion in the tissue fluid starts (Step S5). Next, it is judged whether or not end of the extraction time set up in Step S2 is notified by the alarm 142 of the timer unit 140 (Step S6). In a case where it is notified, the collection member 400 is removed and the tissue fluid extraction is finished (Step S7). Next, the collection member 400 already finishing extraction is installed in the installation unit 205 of the measurement device 200, the tissue fluid measurement and the blood glucose AUC analysis are carried out (Step S8), and measurement ends.

Hereinafter, respective processes are explained in detail.

(Step S1: Preprocessing process)

First, the subject cleans skin 600 with alcohol and the like for removing objects (sweat, dust, etc.) to be a disturbing factor for measurement results.

(Step S2: Timer setting process)

Next, the subject sets up time (extraction time) of the timer unit 140 by operating the decision button 161 and the select/manner button 162. The setup time is set at, for example, "3:00" (3 hours). It may be inputted and set up as 180 minutes. In a case where the display section 160 of the timer unit 140 is caused to display end time, time is set up or confirmed as needed.

Now, with respect to "specific time" in the present invention, time measurement starts with the puncture action and ends with time passage of set time (e.g. 3 hours). On the other hand, "extraction time" is a range from time when the subject applies the collection member 400 as an extraction medium on the skin after puncture to time when the subject who is notified that the set time has passed removes the collection member 400 from the skin 600. Strictly, "extraction time" does not correspond with "specific time". However, time from puncture action to application of the collection member 400 and time from notification of lapse of set time to removal of the collection member 400 are both as short as about dozen seconds to 30 seconds, and both times offset by each other . In view of these, there is no problem to consider that "specific time" set up in the timer unit 140 substantially corresponds with "extraction time" when the tissue fluid is extracted.

Next, the timer unit 140 is mounted on the main body 1a. This mount releases the lock mechanism as described above and the array chuck 40 can be mounted. Then, the fine needle chip 110 is mounted on the array chuck 40, and the array chuck 40 is pushed in depth direction up to the position that the array chuck 40 can be ejected, so that the power supply of the display section 160 is turned on.

(Step S3: Puncture process)

Subsequently, a contact surface 32 of the chip accommodation tool insertion member 30 is applied to the subject's skin which is cleaned, the button section 64 of the release button 60 is pressed, and the array chuck 40 is ejected in a direction of puncture. Subsequently, fine pores 601 are formed on the subject's skin 600 by the fine needle 113a of the fine needle chip 110.

(Steps S4 to S7: Collection member application process to extraction - Accumulation process end)

Next, as shown in FIG. 29, the subject removes a peel-off paper 404 (Refer to FIG. 27) of the collection member 400 and applies the collection member 400 to the site where the fine pores 601 are formed (Step S4). Thus, a gel 401 contacts with the site where the fine pores 601 are formed, and the tissue fluid containing glucose and electrolyte (NaCl) begins to move to the gel 401 through the fine pores 601, and the extraction starts. The state in which the collection member 400 is applied to the skin 600 is kept until the specific time (setup time of the alarm) passes (Step S5). Then, it is judged whether or not the extraction time set up in Step S2 has passed and notifying by voice or vibration is made by the alarm 142 of the timer unit 140 (Step S6). In a case where the notification is made, the subject removes the skin 600 of the collection member 400 according to this notification. Here, since the alarm of the timer unit 140 is set up to 180 minutes, the tissue fluid is continuously extracted from the skin spending 180 minutes. Thereby, the extraction-accumulation process ends (Step S7).

(Step S8: Measurement process)

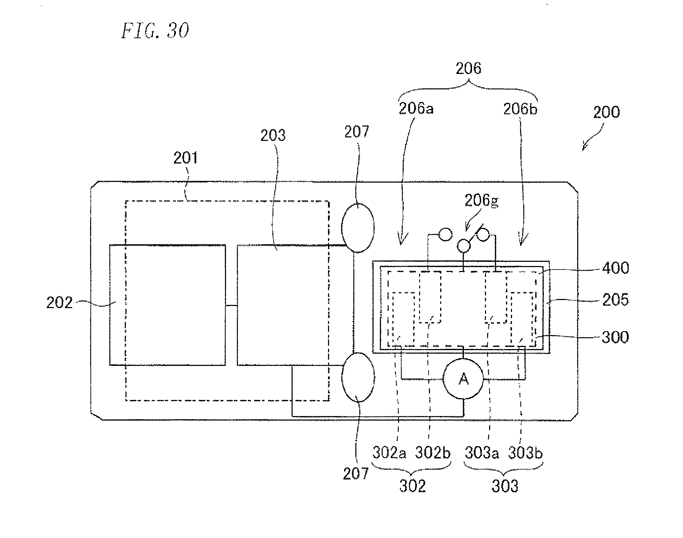

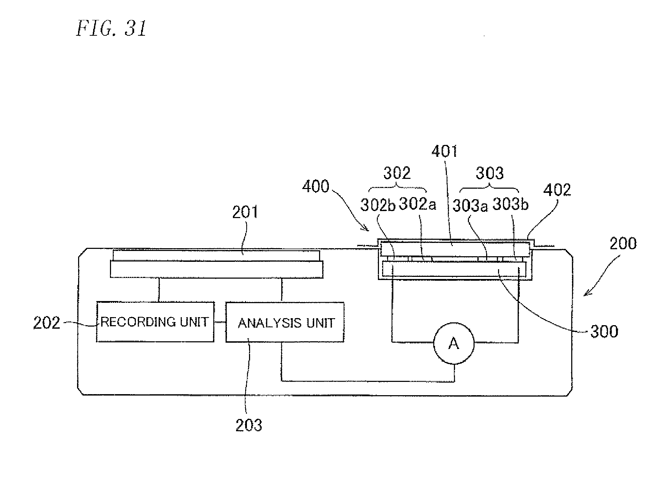

Next, as shown in FIGs. 30 and 31, the subject installs a sensor chip 300 in the installation unit 205 of the measurement device 200 and installs the collection member 400 in a state after finishing the extraction on the sensor chip 300. Thus, a first circuit is configured by a glucose concentration measurement circuit 206a of the measurement device 200, a glucose concentration measurement electrode 302 of the sensor chip 300, and a gel 401 of the collection member 400. A second circuit is configured by a sodium ion concentration measurement circuit 206b of the measurement device 200, a sodium ion concentration measurement electrode 303 of the sensor chip 300, and the gel 401 of the collection member 400.

In a case where concentration of the extracted glucose is measured, the subject switches a switch 206g to the glucose concentration measurement circuit 206a by an operation button 207 and instructs start of measurement. Thus, a constant voltage of specific value is applied to the first circuit through a constant voltage control circuit, and a current value $I_{Glc}$ detected by an ammeter is inputted in an analysis unit 203. Here, the following formula (1) is established between the current value ($I_{Glc}$) and the glucose concentration ($C_{Glc}$) of the gel 401.

$$C_{Glc} = A \cdot I_{Glc} + B \text{ (A and B are constant numbers) ... (1)}$$

The analysis unit 203 calculates the glucose concentration $C_{Glc}$ from the current value $I_{Glc}$ based on the formula (1).

Further, the analysis unit 203 calculates an extraction glucose quantity ($M_{Glc}$) using thus obtained glucose concentration $C_{Glc}$, and extraction solvent quantity or a gel volume V (constant number) based on the following formula (2).

$$M_{Glc} = C_{Glc} \cdot V \dots (2)$$

Further, in a case where an extracted sodium ion concentration is measured, the subject switches a switch 206g to the sodium ion concentration measurement circuit 206b by the operation button 207 and instructs start of measurement. Thus, a voltage value $V_{Na}$ is detected by a voltmeter which is connected in parallel with the second circuit and inputted in the analysis unit 203. Here, the following formula (3) is established between the voltage value $V_{Na}$ and a sodium ion concentration $C_{Na}$ of the gel 401.

$$C_{Na} = C \cdot V_{Na} + D \text{ (C and D are constant numbers)} \dots (3)$$

The analysis unit 203 calculates the sodium ion concentration $C_{Na}$ from the voltage value $V_{Na}$ based on the formula (3).

Further, the analysis unit 203 calculates an extraction rate $J_{Na}$ of sodium ion at the extraction site from the sodium ion concentration $C_{Na}$, a volume V of the gel 401, and extraction time t based on the following formula (4).

$$J_{Na} = C_{Na} \cdot V \cdot 1/t \dots (4)$$

Then, the analysis unit 203 calculates predicted glucose permeability ($P_{Glc}(calc)$) indicative of easiness of glucose extraction from thus calculated sodium ion extraction rate $J_{Na}$ based on the following formula (5).

$$P_{Glc}(calc) = E \cdot J_{Na} + F \text{ (E and F are constant numbers)}$$

$$\dots (5)$$

The formula (5) is obtained as follows.

The glucose permeability indicative of easiness of glucose extraction is originally given by a ratio (this ratio is tentatively referred to as true glucose permeability $P \cdot_{Glc}$) of the blood glucose AUC obtained by blood sampling to an extracted glucose quantity. As described below, because the true glucose permeability $P \cdot_{Glc}$ indicates constant correlation with the sodium ion extraction rate $J_{Na}$, an approximation formula is obtained based on the sodium ion extraction rate $J_{Na}$ and the true glucose permeability $P \cdot_{Glc}$, and the formula (5) can be obtained.

According to the formula (5), it is possible to obtain the predicted glucose permeability $P_{Glc}(calc)$ indicative of easiness of glucose extraction based on sodium ion extraction rate $J_{Na}$ obtainable without blood sampling.

The analysis unit 203 calculates predicted blood glucose AUC (predicted $AUC_{BG}$) from the extraction glucose quantity $M_{Glc}$ obtained by the formula (2) and the predicted glucose permeability $P_{Glc}(calc)$ obtained by the formula (5), based on the following formula (6).

$$\text{predicted } AUC_{BG} = M_{Glc} / P_{Glc}(calc) \dots (6)$$

This predicted blood glucose AUC (predicated $AUC_{BG}$) is a value having high correlation with the blood sampling glucose AUC which is calculated by plural times of blood sampling. A value of the predicated blood glucose AUC is displayed on the display unit 201 and recorded in the recording unit 202. Thus, the measurement process ends.

[Example 2 of Blood glucose AUC measurement]

According to Example 1, the gel 401 in which the tissue fluid extracted from biological body is accumulated in the installation unit 205 of the measurement device 200 is installed, and the glucose concentration and the sodium ion concentration in the gel 401 are measured. However, an analyte in the gel 401 is collected in the pure water and an analyte concentration in thus collected fluid may be measured.

For example, a gel reservoir 420 (the gel 401 is arranged on one surface of a tape member 421) having the gel 401 which finishes extraction of analyte from the skin is set in a collection cartridge 450 for exclusive use. This collection cartridge 450 is formed of a cartridge main body 451 in a box shape, an inlet 452 of the collection fluid is formed on one of facing wall surfaces of the cartridge main body 451, and an outlet 453 of the collection fluid is formed on the other. The gel reservoir 420 is set in the collection cartridge 450 so that the gel 401 projects into the cartridge main body 451 from an opening 454 formed on one surface of the cartridge main body 451.

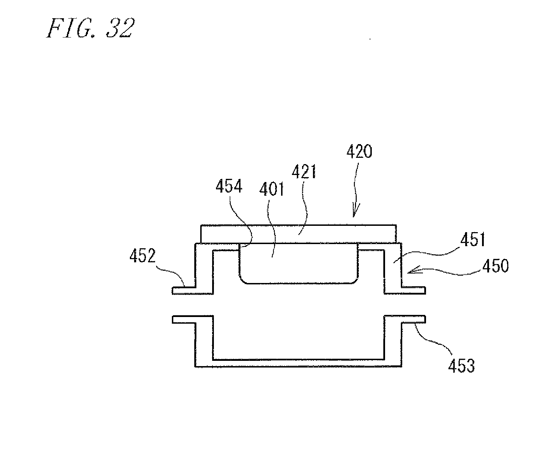

Next, as shown in FIG. 33, the collection cartridge 450 is set in the specific place of a measurement device 460. This measurement device 460 includes a tank unit 461 and a pump unit 462 and a flow passage of collection liquid is formed from the tank unit 461 to a measurement unit 463 through the pump unit 462 and the cartridge main body 451. Further, glucose concentration measurement electrodes 464 and sodium ion concentration measurement electrodes 465 are arranged in the measurement unit 463 similarly to the above-described measurement device 200. After setting of the collection cartridge 450, a collection liquid 469 for collecting the analyte in the gel which is accommodated in the tank unit 461 is transferred into the cartridge main body 451 by driving the pump unit 462 (Refer to FIG. 32). Further, although illustration is omitted, a valve is arranged on downstream side of the outlet 453 of the cartridge main body 451, and the valve is closed before the collection liquid 469 is transferred into the cartridge main body 451.

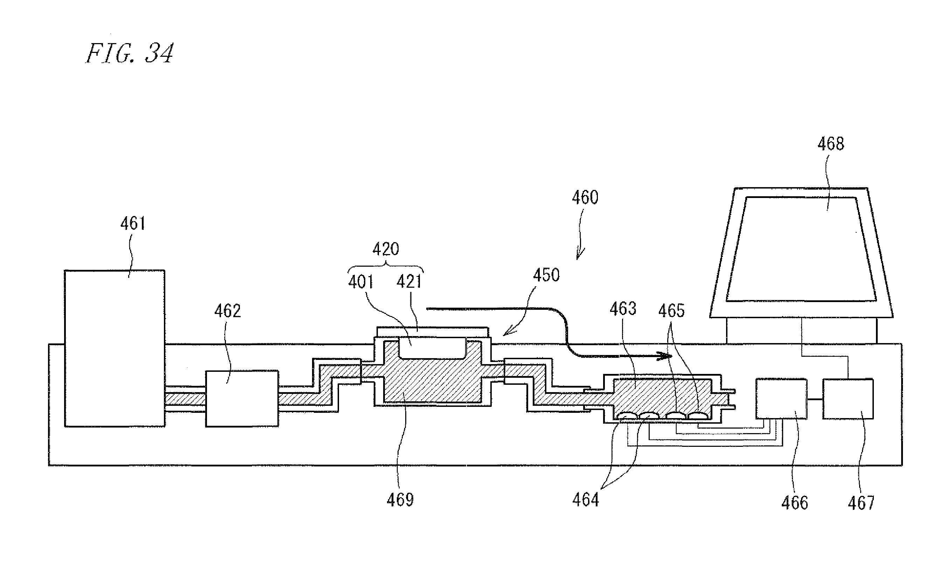

The state of the cartridge main body 451 filled up with the collection liquid 469 is suspended for a given time, and the analyte in the gel 401 is collected in the collection liquid 469. Subsequently, the valve is opened and the collection liquid 469 is transferred to the measurement unit 463 by driving the pump unit 462, as shown in FIG. 34. Next, the glucose concentration and the sodium ion concentration are measured with an electric control unit 466 and an analysis unit 467 by the above-described method using the formulas (1) to (6), and the blood glucose AUC is analyzed. Thus obtained result is outputted on a display unit 468.

In the present embodiment, since the timer unit 140 is removably mounted on the main body 1a, it is possible to separate from the main body 1a the timer unit 140 which is lighter and more compact than the main body 1a and carry it conveniently.

Further, in the present embodiment, there is provided the lock mechanism for inhibiting the puncture action of the puncture mechanism of the main body 1a in a case where the timer unit 140 is removed from the main body 1a. Therefore, it is possible to prevent erroneous operation of starting puncture while forgetting that measurement of extraction time.

Further, in the present embodiment, since the turn-on mechanism is provided for turning on the power supply of the display section 160 by loading the fine needle chip 110 on the array chuck 40 in ejectable condition, there is no need to turn on the power supply of the display section 160 in advance. Therefore, according to the present embodiment, it is possible to save consumption power of the timer unit 140.

Meanwhile the present invention is not limited to the embodiment described before but it is possible to appropriately modify design thereof.

For example, although the timer unit 140 is removable with respect to the main body according to the embodiments described before, the timer unit may be locked on and integrated with the main body. In the embodiment described before, the tip end 157a of the switch section 157 is pressed for turning on the power supply of the display section 160, and the pressure is released by the puncture action and time measurement starts by this release. However, two types of switch sections may be set, in which one of them turns on the power supply of the display section 160 and the other starts time measurement.

Further, although it is described that time is set every 10 minutes in this embodiment, there is no problem for setting every 5 minutes.

Further, in the above-described embodiment, the puncture action is activated by pressure of the button section 64 of the release button 60, and the time measurement by the timer 141 is activated by pressure release of the tip end 157a of the switch section 157 which is caused by the tip end 43d of the array chuck 40. However, the button section 64 may be linked with the tip end 157a of the switch section 157 for starting time measurement by the timer 141, and time measurement by the timer 141 may be activated by the pressure action of the button section 64.

**Claims**

**1.** A puncture device which punctures a skin of a subject for extracting a tissue fluid from a puncture site of the subject, comprising:

a timer;
a notifying section which notifies a user that specific time measured by the timer has passed;
a puncture mechanism which punctures the skin; and
an interlock mechanism which causes the timer to start time measurement in conjunction with a puncture action by the puncture mechanism.

2. The puncture device according to claim 1,
wherein the notifying section comprises at least one of an alarm sound generator and a vibrator.

3. The puncture device according to claim 2, further comprising a notification selecting section for selecting a notifying method by the notifying section.

4. The puncture device according to any one of claims 1 to 3, further comprising:

a main body accommodating the puncture mechanism; and
a timer unit removably mounted on the main body, and accommodating the timer and the notifying section.

5. The puncture device according to claim 4, further comprising a lock mechanism which inhibits the puncture action by the puncture mechanism when the timer unit has been removed from the main body.

6. The puncture device according to claim 5,
wherein the lock mechanism allows the puncture action by the puncture mechanism when the timer unit has been mounted on the main body.

7. The puncture device according to any one of claims 1 to 6, further comprising a press button which is pressed by the user for causing the puncture mechanism to start the puncture action,
wherein the interlock mechanism causes the timer to start the time measurement in conjunction with a pressing action of the press button by the user.

8. The puncture device according to any one of claims 1 to 7, further comprising:

a memory; and
a terminal for transferring from outside to the memory at least one of measurement information on measurement of a specific component in the tissue fluid of the subject and subject information on the subject and for transferring at least one of the measurement information and the subject information which are memorized in the memory to outside.

9. The puncture device according to any one of claims 1 to 8, further comprising a display section which displays at least one of remaining time before the specific time passes and clock time when the specific time passes.

10. The puncture device according to claim 9, further comprising a display selecting section for selecting an object to be displayed on the display section.

11. The puncture device according to claim 9 or 10, further comprising a turn-on mechanism which turns on a power supply of the display section in conjunction with an action of loading on the puncture mechanism a puncture needle for puncturing the skin.

12. An in vivo component measurement system comprising:

a puncture device which punctures a skin of a subject for extracting a tissue fluid from a puncture site of the subject, comprising:

a timer;
a notifying section which notifies a user that time measured by the timer has passed through the specific time;
a puncture mechanism which punctures the skin; and
an interlock mechanism which causes the timer to start time measurement in conjunction with a puncture action by the puncture mechanism;

a collection member for accumulating a specific component in the tissue fluid extracted from the puncture site punctured by the puncture device; and
a measurement device for obtaining a value related to a quantity of the specific component accumulated in the collection member.

13. The in vivo component measurement system according to claim 12,
wherein the specific component is glucose.

14. The in vivo component measurement system according to claim 12 or 13,
wherein the measurement device further comprises an analysis unit which obtains a value corresponding to an area under blood concentration - time curve (AUC) of the specific component during the specific time based on a value related to the specific component.

15. The in vivo component measurement system according to any one of claims 12 to 14,
wherein the collection member comprises an extraction medium for accumulating the specific component in the tissue fluid and a retention member for retaining the extraction medium.

FIG. 1

*FIG. 2*

FIG. 3

FIG. 4

FIG. 5

FIG. 6

**FIG. 7**

FIG. 8

FIG. 9

*FIG. 10*

100

130

I

114

112    111

110

111    112

I

121b

121a    121c

121a    121

121a

122f    120

121c    121a

122b

FIG. 11

*FIG. 12*

FIG. 13

FIG. 14

FIG. 15

FIG. 16

FIG. 17A

FIG. 17B

FIG. 17c

FIG. 17D

*FIG. 18A*

*FIG. 18B*

*FIG. 18C*

FIG. 19

*FIG. 20*

*FIG. 21*

FIG. 22

*FIG. 23*

*FIG. 24*

200

201        205

202 — RECORDING UNIT — ANALYSIS UNIT

206

A

203

FIG. 25

301    300

302b    302a    303a    303b

302    303

FIG. 26

FIG. 27

*FIG. 28*

START

S1 — PREPROCESSING PROCESS

S2 — TIMER SETTING PROCESS

S3 — PUNCTURE PROCESS

S4 — COLLECTION MEMBER APPLICATION PROCESS

S5 — EXTRACTION PROCESS

S5 — HAS SPECIFIC TIME PASSED? — No

Yes

S7 — END OF EXTRACTION AND ACCUMULATION PROCESS

S8 — MEASUREMENT PROCESS

END

*FIG. 29*

FIG. 30

*FIG. 31*

FIG. 32

EP 2 233 068 A1

*FIG. 33*

FIG. 34

EP 2 233 068 A1

FIG. 35

EP 2 233 068 A1

Europäisches
Patentamt

European
Patent Office

Office européen
des brevets

# EUROPEAN SEARCH REPORT

Application Number

EP 10 15 6880

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 891 898 A1 (ROCHE DIAGNOSTICS GMBH [DE]; HOFFMANN LA ROCHE [CH]) 27 February 2008 (2008-02-27) * paragraphs [0003], [0018], [0021] - [0028] * * figures * | 1-3,7-15 | INV. A61B5/00 A61B5/151 |
| A | DE 91 13 046 U1 (FRESE, V.; FRESE-GÖDDEKE, B.) 19 December 1991 (1991-12-19) * page 7, paragraph 7 - page 8, paragraph 3 * * figures * | 1-15 | |
| A,D | US 2007/233011 A1 (HAGINO KEI [JP] ET AL) 4 October 2007 (2007-10-04) * abstract; figures * | 1-15 | |
| A | US 4 627 445 A (GARCIA FERNANDO S [US] ET AL) 9 December 1986 (1986-12-09) * column 6, lines 10-33 * * column 7, line 36 - column 8, line 2 * * figures * | 1-15 | TECHNICAL FIELDS SEARCHED (IPC) A61B |
| A | US 2005/143675 A1 (NEEL GARY T [US] ET AL) 30 June 2005 (2005-06-30) * paragraphs [0044], [0067] * * figures * | 1-15 | |

The present search report has been drawn up for all claims

| Place of search | Date of completion of the search | Examiner |
|---|---|---|
| Berlin | 16 July 2010 | Schultz, Ottmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 15 6880

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2010

| Patent document cited in search report | | Publication date | Patent family member(s) | | Publication date |
|---|---|---|---|---|---|
| EP 1891898 | A1 | 27-02-2008 | US | 2008064986 A1 | 13-03-2008 |
| DE 9113046 | U1 | 19-12-1991 | DE | 4234553 A1 | 22-04-1993 |
| US 2007233011 | A1 | 04-10-2007 | CN | 101036581 A | 19-09-2007 |
| | | | EP | 1834589 A2 | 19-09-2007 |
| | | | JP | 2007236844 A | 20-09-2007 |
| US 4627445 | A | 09-12-1986 | CA | 1277896 C | 18-12-1990 |
| US 2005143675 | A1 | 30-06-2005 | AU | 2004312027 A1 | 21-07-2005 |
| | | | BR | PI0418216 A | 27-04-2007 |
| | | | EP | 1706024 A1 | 04-10-2006 |
| | | | JP | 2007520699 T | 26-07-2007 |
| | | | WO | 2005065539 A1 | 21-07-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070233011 A
- JP 2009063668 W

- US 20070232875 A